# EUROPEAN PATENT APPLICATION

(11) **EP 3 118 202 A1**
(43) Date of publication of application: **18.01.2017**
(21) Application number: 15176937.9
(22) Date of filing: 15.07.2015
(51) Int. Cl.: C07D 471/14, A61K 51/04

(54) **DIHYDROPYRIDOPYRROLE DERIVATIVES AS TAU-PET-LIGANDS**

(71) Applicant: AC Immune S.A., 1015 Lausanne (CH); Piramal Imaging SA, 1753 Matran (CH)
(72) Inventor: The designation of the inventor has not yet been filed
(74) Representative: Vossius & Partner Patentanwälte Rechtsanwälte mbB

(57) **Abstract**

The present invention relates to novel compounds of formula (I) that can be employed in the selective Tau detection of disorders and abnormalities associated with Tau aggregates such as Alzheimer's disease and other Tauopathies using Positron Emission Tomography (PET) Imaging.
Formula (I):

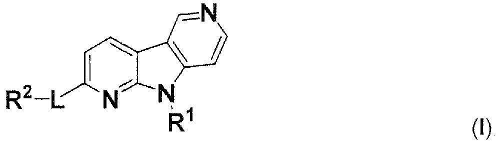

## Description

### Field of the invention

The present invention relates to novel compounds that can be employed in the selective detection of disorders and abnormalities associated with Tau aggregates such as Alzheimer's disease and tauopathies, for example, using Positron Emission Tomography (PET) Imaging.

### Background of the invention

Alzheimer's disease (AD) is a neurological disorder primarily thought to be caused by amyloid plaques, an extracellular accumulation of abnormal deposit of Aβ aggregates in the brain. The other major neuropathological hallmarks in AD are the intracellular neurofibrillary tangles (NFT) that originate by the aggregation of the hyperphosphorylated Tau (Tubulin associated unit) protein, phosphorylated Tau or pathological Tau and its conformers. AD shares this pathology with many neurodegenerative tauopathies, in particularly with specified types of frontotemporal dementia (FTD). In AD brain, Tau pathology (tauopathy) develops later than amyloid pathology, but it is still discussed controversially if amyloid ß protein is the causative agent in AD which constitutes the essence of the so-called amyloid cascade hypothesis (Hardy et al, Science 1992, 256, 184-185, and most recently, Musiek et al, Nature Neurosciences 2015, 18(6), 800-806): "Three dimensions of the amyloid hypothesis: time, space and 'wingmen'".

Presently, the only definite way to diagnose AD is to identify plaques and tangles in brain tissue by histological analysis of biopsy or autopsy materials after the death of the individual.

Therefore, there is a great deal of interest in detection of Tau pathology *in vivo.* Tau PET Imaging promises novel insights into deposition of Tau aggregates in the human brain and might allow to non-invasively examine the degree of Tau pathology, quantify changes in Tau deposition overtime, assess its correlation with cognition and analyze the efficacy of an anti-Tau therapy. For recent reviews see Shah et al, J Nucl Med. 2014, 55(6), 871-874: "Molecular Imaging Insights into Neurodegeneration: Focus on Tau PET Radiotracers" and Ariza et al, J Med Chem 2015, 58(11), 4365-82): "Tau PET Imaging: Past, Present and Future". In addition, several patent applications have recently been published, e.g: WO 2009/102498 and WO 2011/119565 (both Siemens Medical Solutions), WO 2012/067863 and WO 2012/068072 (both GE Healthcare), WO 2015/052105 (Hoffmann-La Roche AG) which claim novel compounds for Tau Imaging.

In order to achieve high target selectivity, molecular probes have been used which recognize and bind to the pathological target. Selectivity for binding to pathological Tau protein over other biological entities is therefore a basic requirement of an imaging probe. In order to reduce background signal interference resulting from non-specific off-target binding (e.g. binding to amyloid-beta or monoamine oxidases), and to reduce dosing requirements, imaging compounds should bind with high affinity to their target. Since amyloid or amyloid-like deposits formed from proteins of diverse primary amino acid sequences share a common β-sheet quaternary conformation, molecular probes are required that can differentiate such structures in order to avoid detection of false-positives and misdiagnosis.

In addition, molecular probes must also be designed such that upon administration they can distribute within the body and reach their target. For imaging of Tau aggregates associated with neurological disorders, such as Alzheimer's disease, imaging compounds are required that can penetrate the blood brain barrier and pass into the relevant regions of the brain. For targeting intracellular Tau aggregates, cell permeability is a further requirement of imaging compounds. A further prerequisite in order to avoid unnecessary accumulation of compound which may result in increased risk of unwanted side-effects is a fast compound wash-out from the brain (or other targeting organ).

The compounds of the present invention demonstrate high affinity to Tau aggregates, high selectivity against other targets in the brain and favorable pharmacokinetic properties.

### Description of the Figure

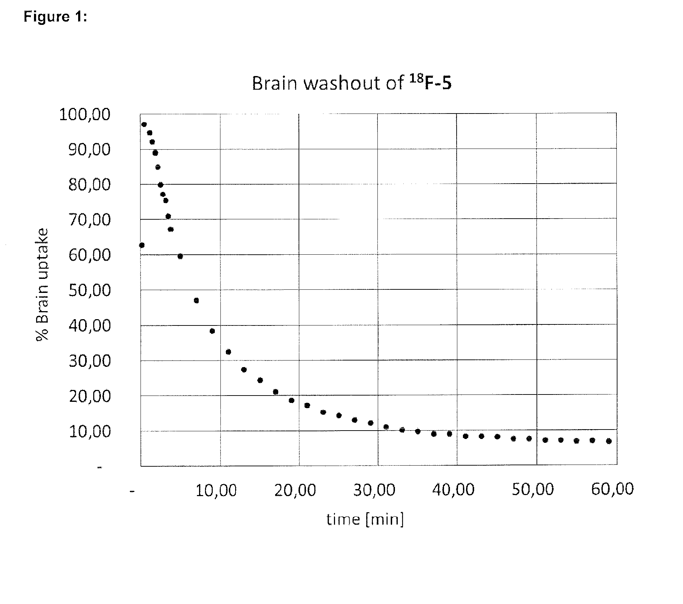

**Figure 1** shows the washout kinetics of the compound **¹⁸F**-**5**.

### Summary of the invention

The present invention relates to a compound of formula **(I):** 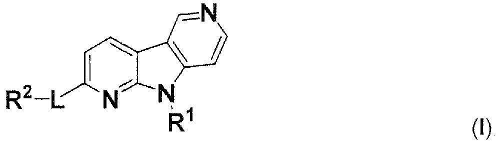 and all stereoisomers, racemic mixtures, pharmaceutically acceptable salts, hydrates, solvates and polymorphs thereof.
**R¹** is -H or -CH₃. In one embodiment, R¹ is -H. In another embodiment, R¹ is -CH₃.
**R²** is fluoro(hydroxy)alkylene. Preferably, the alkylene group has 3 to 6 carbon atoms, more preferably 3 or 4 carbon atoms, even more preferably 3 carbon atoms. The alkylene group can be substituted in any available position by a fluoro atom and a hydroxy group.

An example of a preferred fluoro(hydroxy)alkylene group is 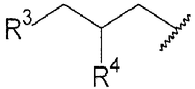 wherein **R**³ is OH or F and wherein **R⁴** is OH or F, with the proviso that if **R³** is OH, **R**⁴ is F, or if **R³** is F, **R**⁴ is OH.

Preferred examples of **R²** include F-CH₂CH(OH)CH₂- and HO-CH₂CH(F)CH₂-.

Another example of a preferred fluoro(hydroxy)alkylene group is 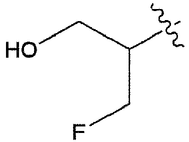 L is 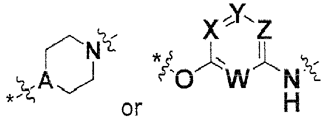 wherein the asterisk indicates the bond to **R².**
**A** is **N,** 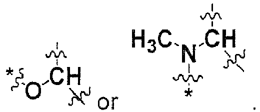 In one embodiment, **A** is N. In another embodiment **A**
is 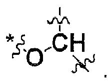 In a further embodiment, A is

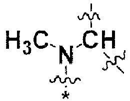

**W, X**, **Y,** and **Z** are selected from CH and N, with the proviso that the ring which includes **W, X**, Y and **Z** contains at most one nitrogen atom.

**F** is [F-19]fluoro or [F-18]fluoro, preferably [F-18]fluoro.

In one embodiment, the invention refers to a compound of formula **(IA):** 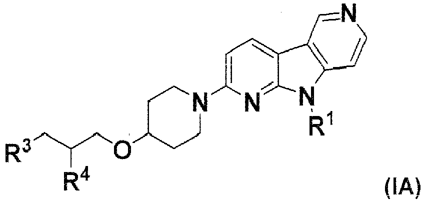 wherein **R**³ is OH or F and wherein **R⁴** is OH or F, with the proviso that if **R³** is OH, **R⁴** is F, or if **R³** is F, **R**⁴ is OH.

In another embodiment, the invention refers to a compound of formula **(IB):** 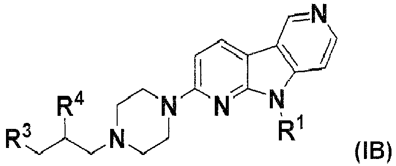 wherein **R**³ is OH or F and wherein **R⁴** is OH or F, with the proviso that if **R³** is OH, **R**⁴ is F, or if **R³** is F, **R**⁴ is OH.

In yet another embodiment, the invention refers to a compound of formula **(IC):** 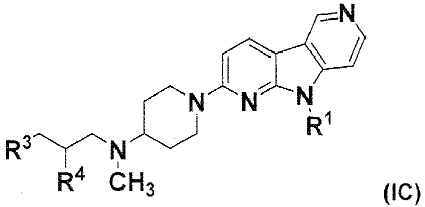 wherein **R**³ is OH or F and wherein **R⁴** is OH or F, with the proviso that if **R**³ is OH, **R**⁴ is F, or if **R**³ is F, **R**⁴ is OH.

In a further embodiment, the invention refers to a compound of formula (ID): 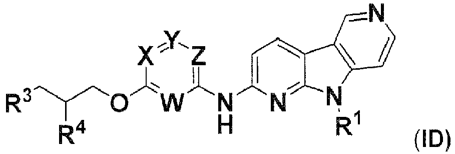 wherein **R**³ is OH or F and wherein **R⁴** is OH or F, with the proviso that if **R³** is OH, **R⁴** is F, or if **R**³ is F, **R⁴** is OH.

### Examples thereof include

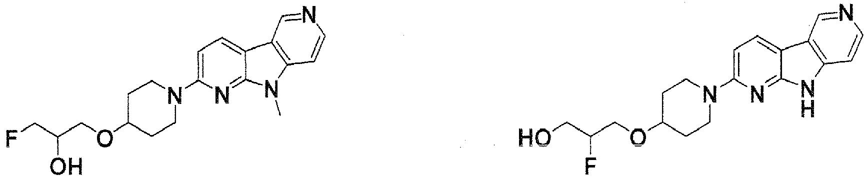

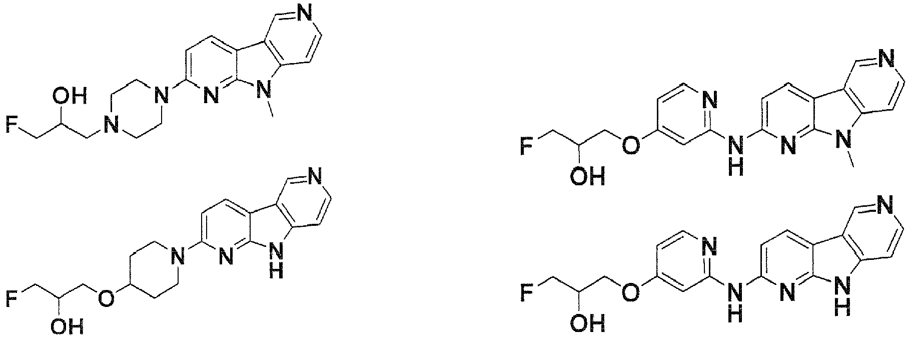

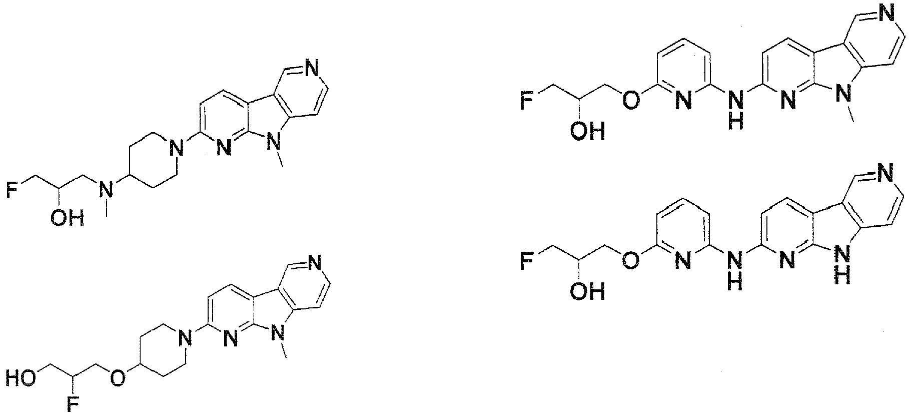

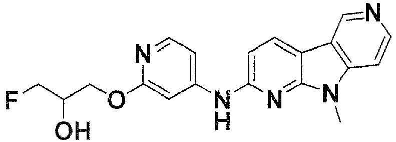

The present invention also refers to a compound of formula **(II):** 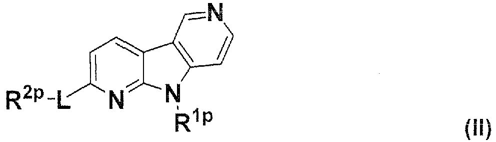 and all stereoisomers, racemic mixtures, pharmaceutically acceptable salts, hydrates, solvates and polymorphs thereof;
which is a precursor of the compound of formula (I).

**R^{1p}** is -H, -CH₃ or **PG¹,** wherein **PG¹** is an amine protecting group. Examples of the amine protecting group include tert-butyloxycarbonyl (Boc), p-tolylsulfonyl (Tosyl), trimethylsilylethoxycarbonyl (Teoc), carbobenzyloxy (Cbz), p-methoxybenzyl carbonyl (Moz or MeOZ), 9-fluorenylmethyloxycarbonyl (Fmoc), acetyl (Ac), or trifluoroacetyl. Preferred examples are (CH₃)₃C-OCO- and CH₃C₆H₄-SO₂-. In one embodiment, **R^{1p}** is -H. In another embodiment, **R^{1p}** is -CH₃. In another embodiment, **R^{1p}** is **PG**¹.

**R^{2p}** is **LG**(O**PG**²)alkylene. Preferably, the alkylene group has 3 to 6 carbon atoms, more preferably 3 or 4 carbon atoms, even more preferably 3 carbon atoms. The alkylene group is substituted in any available position by a leaving group **LG** and a protected hydroxy group (O**PG**²).

An example of a preferred **LG**(O**PG**²)alkylene group is 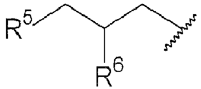 wherein **R⁵** is **LG** or O**PG²** and wherein **R⁶** is O**PG²** or **LG,** with the proviso that if **R⁵** is O**PG², R⁶** is **LG,** or if **R⁵** is **LG, R⁶** is O**PG².**

In a preferred embodiment **R^{2p}** is **LG**-CH₂CH(O**PG²**)CH₂- or **PG**²O-CH₂CH(**LG**)CH₂-.

Another example of a preferred **LG**(O**PG**²)alkylene group is

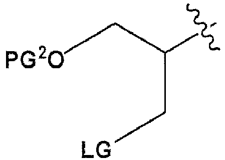

L, A, **X**, Y, **Z** and **W** are as defined above.

**PG²** is a hydroxy protecting group, preferably

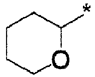

**LG** is a leaving group such as halogen (e.g., Br, I or Cl) or a sulfonate leaving group. Preferably, sulfonate leaving group is CH₃C₆H₄-SO₂-O- or CH₃SO₂-O-.

In one preferred embodiment, the present invention relates to compounds of formula **(IIA):** 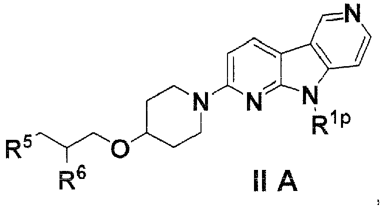 wherein **R⁵** is O**PG**² or LG and wherein **R⁶** is O**PG**² or **LG,** with the proviso if **R⁵** is O**PG²**,**R⁶** is **LG,** or if **R⁵** is **LG, R⁶** is O**PG²**.

In another embodiment, the present invention relates to compounds of formula **(IIB)**: 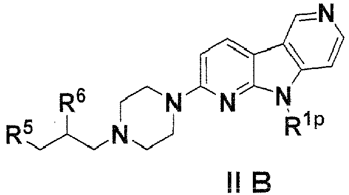 wherein **R⁵** is O**PG²** or **LG** and wherein **R**⁶ is **OPG²** or **LG,** with the proviso if **R⁵** is O**PG²**,**R⁶** is **LG,** or if **R⁵** is **LG, R⁶** is O**PG²**.

In another embodiment, the present invention relates to compounds of formula **(IIC):** 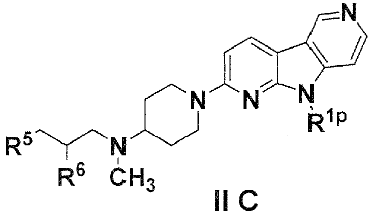 wherein **R⁵** is O**PG**² or **LG** and wherein **R**⁶ is O**PG**² or **LG,** with the proviso if **R**⁵ is O**PG**², **R**⁶ is **LG,** or if **R**⁵ is **LG, R**⁶ is O**PG².**

In another preferred embodiment, the present invention relates to compounds of formula (**IID**): 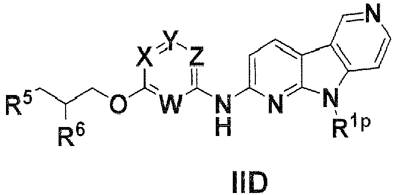 wherein **R**⁵ is O**PG²** or **LG** and wherein **R**⁶ is O**PG**² or **LG,** with the proviso if **R**⁵ is O**PG**²,**R**⁶ is **LG,** or if **R**⁵ is **LG, R**⁶ is O**PG².**

Examples thereof include

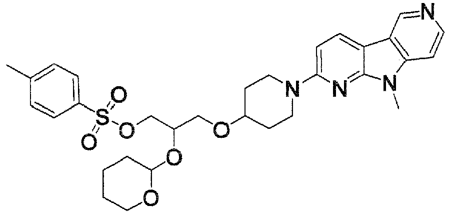

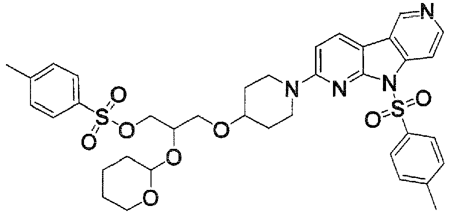

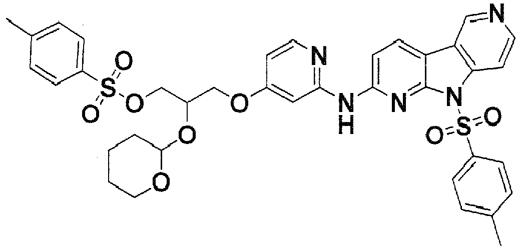

It is understood that all combinations of the above mentioned embodiments are also envisaged.

### Definitions

Within the meaning of the present application the following definitions apply:

"Alkyl" refers to a saturated straight or branched organic moiety consisting of carbon and hydrogen atoms. Examples of suitable alkyl groups have 1 to 6 carbon atoms, preferably 1 to 4 carbon atoms, and include methyl, ethyl, propyl, isopropyl, n-butyl, t-butyl and isobutyl.

"Alkylene" refers to a divalent saturated, linear or branched organic moiety consisting of carbon and hydrogen atoms. Examples of suitable alkylene groups have 1 to 6 carbon atoms, preferably 1 to 4 carbon atoms, and include methylene, ethylene, propylene, isopropylene, n-butylene, t-butylene and isobutylene.

"Aryl" refers to homocyclic aromatic organic moieties containing 1 or 2 rings consisting of carbon and hydrogen atoms which preferably have 6 to 12 carbon atoms, more preferably 6 carbon atoms. Examples are, but not limited to, phenol, biphenyl, and naphthyl.

"Alkoxy" refers to the group -O-alkyl or -O-alkylene-.

"Arylalkyl" refers to a group aryl-alkyl or aryl-alkylene-.

An "amine protecting group" is any protecting group which is suitable for protecting an amine group during an envisaged chemical reaction. Examples of suitable protecting groups are well-known to a person skilled in the art. Suitable protecting groups are discussed, e.g., in the textbook Greene and Wuts, Protecting groups in Organic Synthesis, third edition, page 494-653, which is included herein by reference. Specific preferred examples of the amine-protecting groups include tert-butyloxycarbonyl (Boc), p-tolylsulfonyl (Tosyl), trimethylsilylethoxycarbonyl (Teoc), carbobenzyloxy (Cbz), p-methoxybenzyl carbonyl (Moz or MeOZ), 9-fluorenylmethyloxycarbonyl (Fmoc), acetyl (Ac), or trifluoroacetyl.

A "hydroxy protecting group" is any protecting group which is suitable for protecting a hydroxy group during an envisaged chemical reaction. Examples of suitable protecting groups are well-known to a person skilled in the art. Examples thereof include but are not limited to ethers and silyl ethers. Suitable protecting groups are discussed, e.g., in the textbook Greene and Wuts, Protecting groups in Organic Synthesis, third edition, page 23-148, which is included herein by reference. Specific preferred examples of the hydroxy-protecting groups include methoxymethyl (MOM), 2-methoxyethoxymethyl (MEM), 2-(trimethylsilyl)ethoxymethyl (SEM), tetrahydropyranyl (THP), triisopropylsilyl (TIPS), *t-*butyldimethylsilyl (TBDMS), or *t*-butyldiphenylsilyl (TBDPS).

A "leaving group" is any leaving group which can be attached to a saturated carbon atom and means an atom or group of atoms can be replaced by another atom or group of atoms. Examples are given e.g. in Synthesis (1982), 85-125, table 2 (p. 86; (the last entry of this table 2 needs to be corrected: "n-C₄F₉S(O)₂-O- nonaflat" instead of "n-C₄H₉S(O)₂-O-nonaflat"), Carey and Sundberg, Organische Synthese, (1995), 279-281, table 5.8; or Netscher, Recent Res. Dev. Org. Chem., 2003, 7, 71-83, schemes 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pages 15 to 50, particularly scheme 4 page 25, scheme 5 page 28, table 4 page 30, Figure 7 page 33). Preferably, the "leaving group" is halogen (e.g., Br, I, Cl), an alkyl sulfonate leaving group, or an aryl sulfonate leaving group.

An "alkyl sulfonate leaving group" includes any group which comprises a -OSO₂-R group, with R being selected from the group consisting of C₁₋₄ alkyl and perfluoro(C₁₋₄)alkyl. This includes but is not limited to methyl sulfonyloxy, trifluoromethyl sulfonyloxy and nonafluorobutyl sulfonyloxy.

An "aryl sulfonate leaving group" includes any group which comprises a -OSO₂-R group, with R being selected from the group consisting of aryl which can be optionally substituted by C₁₋₄ alkyl, halogen and nitro. This includes but is not limited to (4-methylphenyl) sulfonyloxy, (4-bromo-phenyl)sulfonyloxy, (4-nitro-phenyl) sulfonyloxy, (2-nitro-phenyl) sulfonyloxy, (4-isopropyl-phenyl) sulfonyloxy, (2,4,6-tri-isopropyl-phenyl) sulfonyloxy, (2,4,6-trimethyl-phenyl) sulfonyloxy, (4-tert-butyl-phenyl) sulfonyloxy and (4-methoxy-phenyl) sulfonyloxy.

In a more preferred embodiment, "aryl sulfonate leaving group" is methyl sulfonyloxy, (4-methylphenyl) sulfonyloxy, or trifluoromethyl sulfonyloxy.

Compounds of the present invention having one or more optically active carbons can exist as racemates and racemic mixtures, stereoisomers (including diastereomeric mixtures and individual diastereomers, enantiomeric mixtures and single enantiomers, mixtures of conformers and single conformers). The term "stereoisomers and mixtures thereof" is intended to mean racemates and racemic mixtures, stereoisomers (including diastereomeric mixtures and individual diastereomers, enantiomeric mixtures and single enantiomers, mixtures of conformers and single conformers). The mixtures of stereoisomers can be optically active or non-optically active. All isomeric forms are included in the present invention. Also included in this invention are all salt forms, polymorphs, hydrates and solvates.

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The patients or subjects in the present invention are typically animals, particularly mammals, more particularly humans.

Tau as used herein refers to a highly soluble microtubule binding protein mostly found in neurons and includes the major 6 isoforms, cleaved or truncated forms, and other modified forms such as arising from phosphorylation, glycosylation, glycation, prolyl isomerization, nitration, acetylation, polyamination, ubiquitination, sumoylation and oxidation.

Neurofibrillary Tangles (NFTs) as used herein refer to insoluble aggregates of the hyperphosphorylated tau protein containing paired helical filaments and straight filaments. Their presence is a hallmark of AD and other diseases known as tauopathies.

The preferred definitions given in the "Definition"-section apply to all of the embodiments described below unless stated otherwise.

### Diagnostic procedures

The compounds of the present invention are suitable for imaging of neurodegenerative disorders. The compounds of the present invention are particularly suitable for imaging of tau protein aggregates. With respect to tau protein, the compounds of the present invention are able to bind to pathologically aggregated tau, hyperphosphorylated tau, neurofibrillary tangles, paired helical filaments, straight filaments, neurotoxic soluble oligomers, polymers and fibrils. The compounds of the present invention are particularly suitable for binding to various types of tau aggregates.

Due to the above binding characteristics, the compounds of the present invention are suitable for use in the diagnosis of disorders associated with tau protein aggregates. The compounds of the present invention are particularly suitable for positron emission tomography imaging of tau deposits.

Diseases involving tau aggregates are generally listed as tauopathies and these include, but are not limited to, Alzheimer's disease (AD), familial AD, Creutzfeldt-Jacob disease, dementia pugilistica, Down's Syndrome, Gerstmann-Straussler-Scheinker disease, inclusion-body myositis, prion protein cerebral amyloid angiopathy, traumatic brain injury, amyotrophic lateral sclerosis, Parkinsonism-dementia complex of Guam, non-Guamanian motor neuron disease with neurofibrillary tangles, argyrophilic grain disease, corticobasal degeneration, diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease, progressive subcortical gliosis, progressive supranuclear palsy (PSP), subacute sclerosing panencephalitis, tangle only dementia, postencephalitic Parkinsonism, myotonic dystrophy, tau panencephalopathy, AD-like with astrocytes, certain prion diseases (GSS with tau), mutations in LRRK2, Hallervorden-Spatz disease, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, and white matter tauopathy with globular glial inclusions, etc (Williams et al., Intern. Med. J., 2006, 36, 652-60).

Due to their design the compounds of the present invention are particularly suitable for use in the diagnosis of Alzheimer's disease, as well as other neurodegenerative tauopathies such as Creutzfeldt-Jacob disease, dementia pugilistica, amyotrophic lateral sclerosis, argyrophilic grain disease, corticobasal degeneration, frontotemporal dementia with Parkinsonism linked to chromosome 17, Pick's disease, progressive supranuclear palsy (PSP), tangle only dementia, Parkinson dementia complex of Guam, Hallervorden-Spatz disease and fronto-temporal lobar degeneration.

In the methods of diagnosing a disorder associated with tau protein aggregates such as Alzheimer's disease, or a predisposition therefor in a subject, the method comprising:
a) administering to the mammal a diagnostically effective amount of a compound of the present invention;
b) allowing the compound of the present invention to distribute into the tissue of interest (such as brain tissue or body fluids such as cerebrospinal fluid (CSF)); and
c) imaging the tissue of interest, wherein an increase in binding of the compound of the present invention to the tissue of interest compared to a normal control level of binding indicates that the subject is suffering from or is at risk of developing a disorder associated with tau protein aggregates.

The compounds of the present invention can be used for imaging of tau protein aggregates in any sample or a specific body part or body area of a patient which suspected to contain a tau protein aggregate. The compounds of the present invention are able to pass the blood-brain barrier. Consequently, they are particularly suitable for imaging of tau protein aggregates in the brain, as well as in body fluids such as cerebrospinal fluid (CSF).

In diagnostic applications, the compound of the present invention is preferably administered in a diagnostic composition comprising the compound of the invention. A "diagnostic composition" is defined in the present invention as a composition comprising compounds of the present invention in a form suitable for administration to mammals such as humans. Preferably a diagnostic composition further comprises a physiologically acceptable carrier, diluent, adjuvant or excipient. Administration is preferably carried out by injection of the composition as an aqueous solution. Such a composition may optionally contain further ingredients such as solvents (including ethanol), buffers; pharmaceutically acceptable solubilisers (e.g., cyclodextrins or surfactants such as Pluronic, Tween or phospholipids); and pharmaceutically acceptable stabilisers or antioxidants (such as ascorbic acid, gentisic acid or para-aminobenzoic acid). The dose of the compound of the present invention will vary depending on the exact compound to be administered, the weight of the patient, and other variables as would be apparent to a physician skilled in the art. Generally, the dose could preferably lie in the range 0.001 µg/kg to 10 µg/kg, preferably 0.01 µg/kg to 1.0 µg/kg. The radioactive dose can be, e.g., 100 to 600 MBq, more preferably 150 to 450 MBq.

Diagnosis of a tau disorder or of a predisposition to a tau-associated disorder in a patient may be achieved by detecting the specific binding of a compound according to the invention to the tau protein aggregates in a sample or *in situ,* which includes:
(a) bringing the sample or a specific body part or body area suspected to contain the tau protein aggregate into contact with a compound of the invention which binds the tau protein aggregate,
(b) allowing the compound of the invention to bind to the tau protein aggregate to form a compound/tau protein aggregate complex (hereinafter "compound/tau protein aggregate complex" will be abbreviated as "compound/protein aggregate complex"),
(c) detecting the formation of the compound/protein complex,
(d) optionally correlating the presence or absence of the compound/protein complex with the presence or absence of tau protein aggregates in the sample or specific body part or area, and
(e) optionally comparing the amount of the compound/protein to a normal control value, wherein an increase in the amount of the compound/protein compared to a normal control value may indicate that the patient is suffering from or is at risk of developing a tau-associated disorder.

After the sample or a specific body part or body area has been brought into contact with the compound of the present invention, the compound is hallowed to bind to the tau protein aggregate. The amount of time required for binding will depend on the type of test (e.g., *in vitro* or *in vivo*) and can be determined by a person skilled in the field by routine experiments.

The compound which has bound to the tau protein aggregate can be subsequently detected by any appropriate method. A preferred method is positron emission tomography (PET).

The presence or absence of the compound/protein is then optionally correlated with the presence or absence of tau protein aggregates in the sample or specific body part or area. Finally, the amount of the compound/protein can be compared to a normal control value which has been determined in a sample or a specific body part or body area of a healthy subject, wherein an increase in the amount of the compound/protein compared to a normal control value may indicate that the patient is suffering from or is at risk of developing a tau-associated disorder.

The present invention also relates to a method of determining the amount of tau protein aggregate in a tissue and/or a body fluid. This method comprises the steps of:
(a) providing a sample representative of the tissue and/or body fluid under investigation;
(b) testing the sample for the presence of tau protein aggregate with a compound of the present invention;
(c) determining the amount of compound bound to the tau protein aggregate; and
(d) calculating the amount of tau protein aggregate in the tissue and/or body fluid.

The sample can be tested for the presence of tau protein aggregate with a compound of the present invention by bringing the sample into contact with a compound of the invention, allowing the compound of the invention to bind to the tau protein aggregate to form a compound/protein aggregate complex and detecting the formation of the compound/protein complex as explained above.

Monitoring minimal residual disorder in a patient suffering from a disorder associated with tau protein aggregates who has been treated with a medicament with a compound according to the invention may be achieved by:
(a) bringing a sample or a specific body part or body area suspected to contain a tau protein aggregate into contact with a compound of the present invention;
(b) allowing the compound to bind to the tau protein aggregate to form a compound/protein aggregate complex;
(c) detecting the formation of the compound/protein aggregate complex;
(d) optionally correlating the presence or absence of the compound/protein aggregate complex with the presence or absence of tau protein aggregate in the sample or specific body part or body area; and
(e) optionally comparing the amount of the compound/protein aggregate to a normal control value, wherein an increase in the amount of the aggregate compared to a normal control value may indicate that the patient may still suffer from a minimal residual disease.

How steps (a) to (e) can be conducted has already been explained above.

Predicting responsiveness of a patient suffering from a disorder associated with tau protein aggregates and being treated with a medicament can be achieved by
(a) bringing a sample or a specific body part or body area suspected to contain a tau protein aggregate into contact with a compound of the present invention;
(b) allowing the compound to bind to the tau protein aggregate to form a compound/protein aggregate complex;
(c) detecting the formation of the compound/protein aggregate complex;
(d) optionally correlating the presence or absence of the compound/protein aggregate complex with the presence or absence of tau protein aggregate in the sample or specific body part or body area; and
(e) optionally comparing the amount of the compound/protein aggregate to a normal control value.

How steps (a) to (e) can be conducted has already been explained above.

In the method for predicting responsiveness the amount of the compound/protein complex can be optionally compared at various points of time during the treatment, for instance, before and after onset of the treatment or at various points of time after the onset of the treatment. A change, especially a decrease, in the amount of the compound/protein complex may indicate that the patient has a high potential of being responsive to the respective treatment.

A compound according to the present invention can also be incorporated into a test kit for detecting a tau protein aggregate. The test kit typically comprises a container holding one or more compounds according to the present invention and instructions for using the compound for the purpose of binding to a tau protein aggregate to form a compound/protein complex and detecting the formation of the compound/protein complex such that presence or absence of the compound/protein complex correlates with the presence or absence of the tau protein aggregates.

The term "test kit" refers in general to any diagnostic kit known in the art. More specifically, the latter term refers to a diagnostic kit as described in Zrein et al., Clin. Diagn. Lab. Immunol., 1998, 5, 45-49.

### Diagnostic compositions

The compounds of the present invention can be used in diagnosis of neurodegenerative disorders, especially disorders associated with Tau protein aggregates.

The invention also provides a diagnostic composition which comprises an effective amount of a compound of formulae (I) in admixture with a pharmaceutically acceptable carrier, diluent, adjuvant or excipient, suitable for administration to mammals such as humans.

Pharmaceutically acceptable excipients are well known in the pharmaceutical art, and are described, for example, in Remington's Pharmaceutical Sciences, 15th Ed., Mack Publishing Co., New Jersey (1975). The pharmaceutical excipient can be selected with regard to the intended route of administration and standard pharmaceutical practice. The excipient must be acceptable in the sense of being not deleterious to the recipient thereof.

Pharmaceutically useful excipients that may be used in the formulation of the diagnostic composition of the present invention may comprise, for example, carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate, binders, adjuvants, solubilizers, thickening agents, stabilizers, disintegrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium stearate, talc, monosaccharides, disaccharides, starch, gelatin, cellulose, methylcellulose, sodium carboxymethyl cellulose, dextrose, hydroxypropyl-ß-cyclodextrin, polyvinylpyrrolidone, low melting waxes, and ion exchange resins.

If the compounds of the present invention are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile aqueous solution which may contain other excipients. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

A proposed dose of the compounds according to the present invention for administration to a human is in the range of 100-600 MBq of a fluorine-18 labeled compound of the present invention, preferably in the range of 150-450 MBq.

The pharmaceutical compositions of the invention can be produced in a manner known per se to the skilled person as described, for example, in Remington's Pharmaceutical Sciences, 15th Ed., Mack Publishing Co., New Jersey (1975).

Diseases that can be detected and monitored with the compounds of the present invention can be associated with the formation of abnormal protein structures, in particular abnormal beta-sheet structures. In the context of the present invention, an abnormal protein structure is a protein structure that arises when a protein or peptide refolds from its natural occurring conformation in healthy individuals, into a beta-sheet three-dimensional structure, which is associated with a pathological condition. Likewise, an abnormal beta-sheet structure in the context of the present invention is a beta-sheet structure that arises when a protein or peptide refolds from its natural occurring conformation in healthy individuals, into a different beta-sheet structure, which is associated with a pathological condition.

In particular, in one embodiment diseases or disorders that can be detected and monitored with the compounds of the present invention are diseases or conditions associated tau proteins aggregates.

The diseases or conditions that can be detected and monitored with the compounds of the present invention include neurodegenerative disorders such as tauopathies. Examples of diseases and conditions which can be detected and monitored are caused by or associated with the formation of neurofibrillary lesions. This is the predominant brain pathology in tauopathy. The diseases and conditions comprise a heterogeneous group of neurodegenerative diseases or conditions including diseases or conditions which show coexistence of tau and amyloid pathologies. Examples of the diseases and conditions which can be treated, alleviated or prevented include Alzheimer's disease (AD), Creutzfeldt-Jacob disease, dementia pugilistica, Down's Syndrome, Gerstmann-Sträussler-Scheinker disease, inclusion-body myositis, prion protein cerebral amyloid angiopathy, traumatic brain injury, amyotrophic lateral sclerosis, Parkinsonism-dementia complex of Guam, non-Guamanian motor neuron disease with neurofibrillary tangles, argyrophilic grain disease, corticobasal degeneration, diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease, progressive subcortical gliosis, progressive supranuclear palsy (PSP), subacute sclerosing panencephalitis, tangle only dementia, postencephalitic Parkinsonism, myotonic dystrophy, tau panencephalopathy, AD-like with astrocytes, certain prion diseases (GSS with tau), mutations in LRRK2, Hallervorden-Spatz disease, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, white matter tauopathy with globular glial inclusions, traumatic stress syndrome, epilepsy, Lewy body dementia (LBD), hereditary cerebral hemorrhage with amyloidosis (Dutch type), mild cognitive impairment (MCI), multiple sclerosis, Parkinson's disease, HIV-related dementia, adult onset diabetes, senile cardiac amyloidosis, endocrine tumors, glaucoma, ocular amyloidosis, primary retinal degeneration, macular degeneration (such as age-related macular degeneration (AMD)), optic nerve drusen, optic neuropathy, optic neuritis, lattice dystrophy. Preferably the diseases and conditions which can be treated, alleviated or prevented include Alzheimer's disease, as well as other neurodegenerative tauopathies such as Alzheimer's disease (AD), Creutzfeldt-Jacob disease, dementia pugilistica, amyotrophic lateral sclerosis, argyrophilic grain disease, corticobasal degeneration, frontotemporal dementia with Parkinsonism linked to chromosome 17, Pick's disease, progressive supranuclear palsy (PSP), tangle only dementia, Parkinson dementia complex of Guam, Hallervorden-Spatz disease and fronto-temporal lobar degeneration.

The compounds of the present invention can be synthesized by one of the general methods shown in the following schemes. These methods are only given for illustrative purposes and should not to be construed as limiting.

General synthetic schemes for the preparation of tricyclic building blocks of this invention:

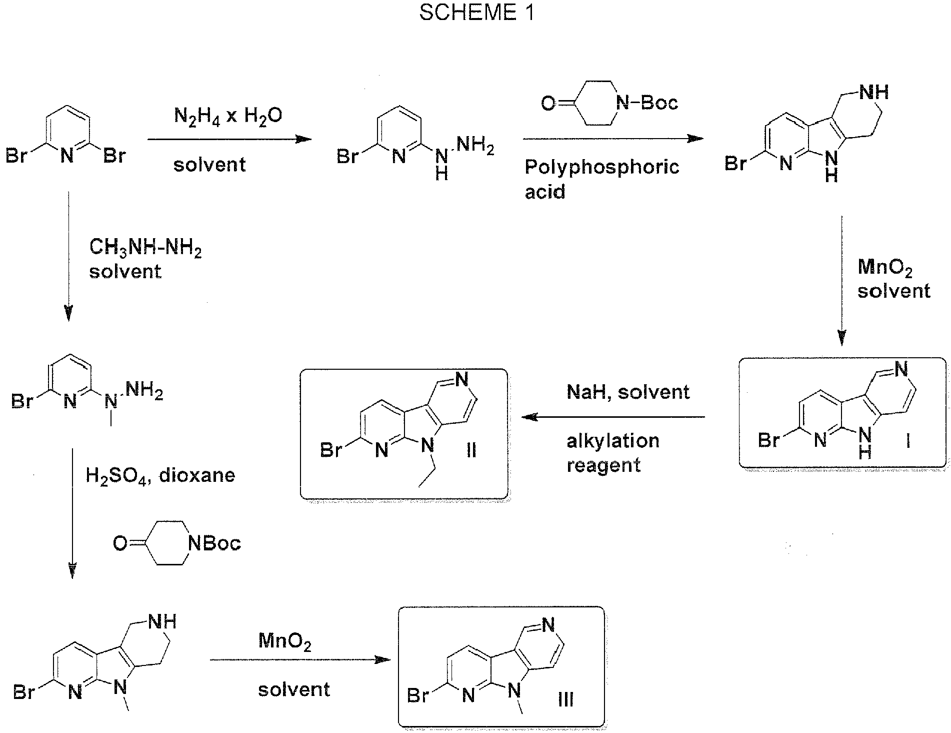

Commercially available 2,6-dibromopyridine was heated under reflux with hydrazine hydrate in a solvent to afford the desired hydrazine-pyridine derivative after purification. The hydrazine-pyridine derivative was then treated with commercially available *N*-Boc-piperidone under the conditions of a Fischer-indole synthesis using polyphosphoric acid to afford the corresponding azaindole derivative as a free base after purification. Oxidation of the azaindole derivative with manganese dioxide in a solvent afforded the desired tricyclic building block I after purification. Alkylation of the NH-moiety with a suitable alkylation agent (CH₃CH₂I, etc.) in a solvent and in the presence of sodium hydride afforded the desired tricyclic building block II after purification. Commercially available 2,6-dibromopyridine was heated under reflux with *N*-methylhydrazine in a solvent to afford the desired *N-*methylhydrazine-pyridine derivative after purification. The *N*-methylhydrazine-pyridine derivative was then treated with commercially available *N*-Boc-piperidone under the conditions of a Fischer-indole synthesis to afford the corresponding azaindole derivative as a free base after purification. Oxidation of the azaindole derivative with manganese dioxide in a solvent afforded the desired tricyclic building block III after purification.

### General synthetic scheme for the preparation of compounds of this invention:

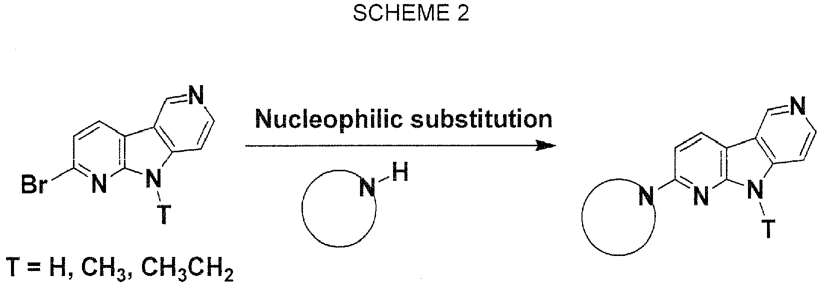

Tricyclic building blocks I, II and III were treated with cycloamine/bicycloamine derivatives containing a fluoro moiety in a solvent using a microwave to enable a nucleophilic substitution reaction to afford the desired final compounds after purification.

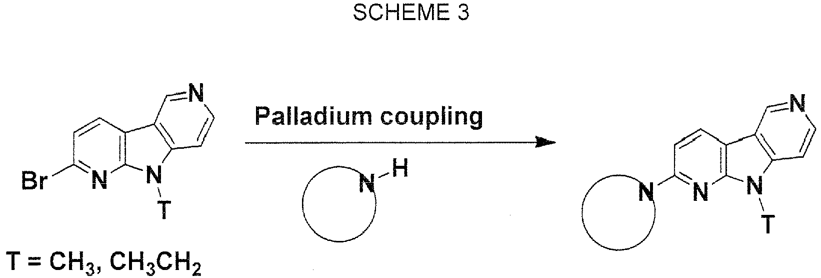

Tricyclic building blocks II and III (Hal = Br) were treated with cycloamine/bicycloamine/ spirocycloamine derivatives containing a fluoro moiety in a solvent via palladium-catalyzed crosscoupling (Buchwald-Hartwig amination) conditions to afford the desired final compounds as solids after purification.

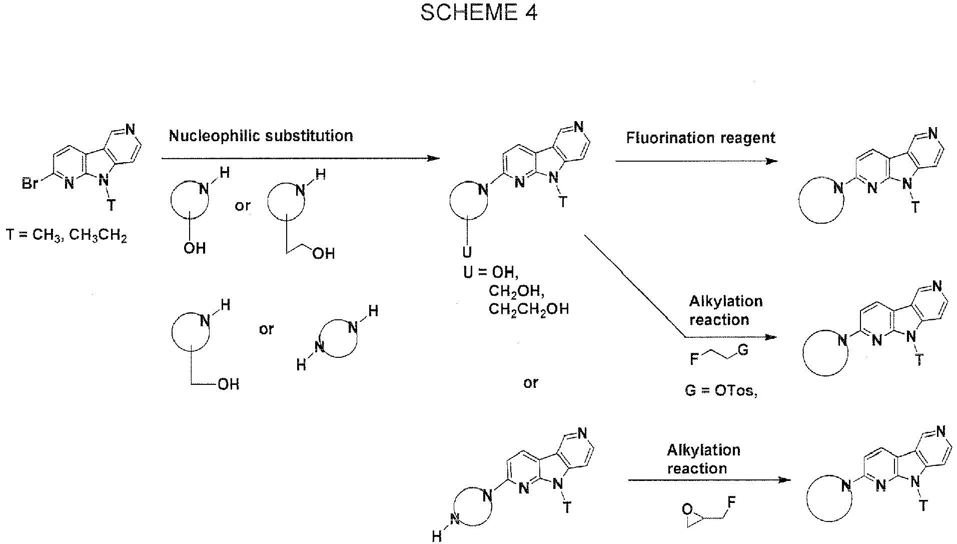

Tricyclic building blocks II and III were treated with cycloamine/bicycloamine derivatives containing a hydroxyl- or amino-moiety in a solvent using a microwave to enable a nucleophilic substitution reaction to afford the desired intermediates after purification. Treatment of intermediates with U = OH, CH₂OH with a fluorination reagent (Deoxo-Fluor, etc.) in a solvent afforded the desired final compounds after purification. Alkylation of intermediates with U = OH with a fluoroalkyl reagent having a suitable leaving group (Br, Tos, etc.) in a solvent afforded the desired final compounds after purification. The Alkylation reaction of piperazine derivatives with a suitable fluoro-oxirane derivative (2-(fluoromethyl)oxirane) in a solvent afforded the desired final compounds after purification.

General synthetic scheme for the preparation of precursor compounds of this invention:

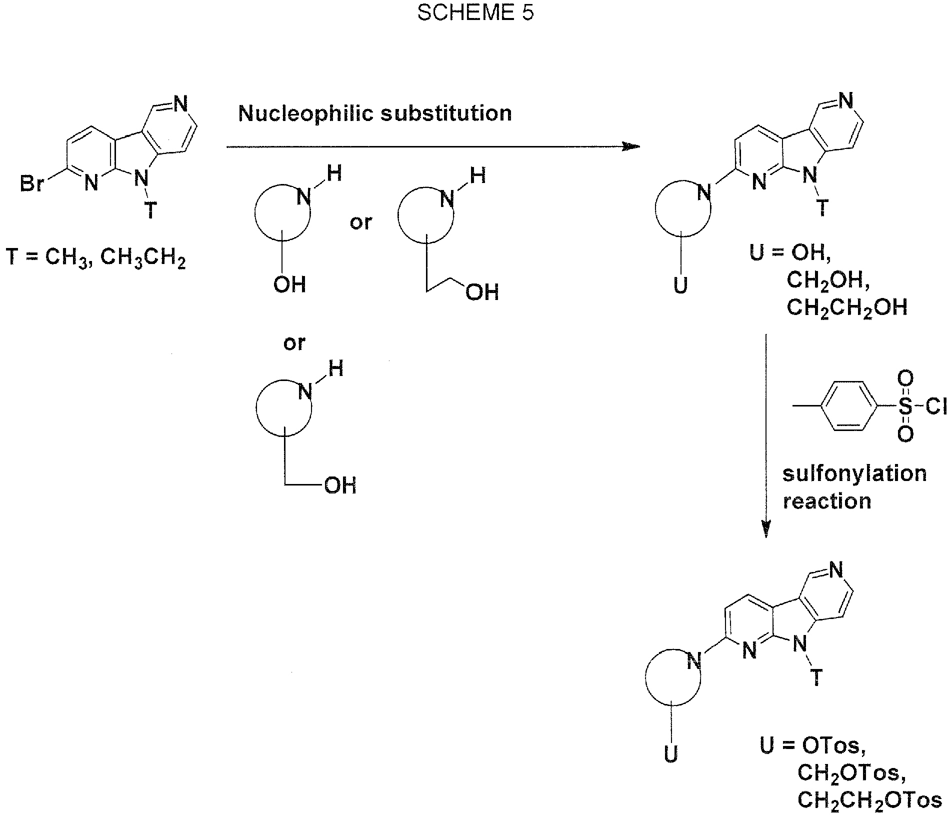

Tricyclic building blocks II and III were treated with cycloamine/bicycloamine derivatives containing a hydroxyl-moiety in a solvent using a microwave to enable a nucleophilic substitution reaction to afford the desired alcohol derivatives after purification. The alcohol derivatives with U = OH, CH₂OH and (CH₂)₂OH were treated with a suitable sulfonylation reagent (CH₃SO₂Cl, p-Tos-Cl) in a solvent to afford the desired precursor compounds to allow the introduction of the ¹⁸F-label in the following step.

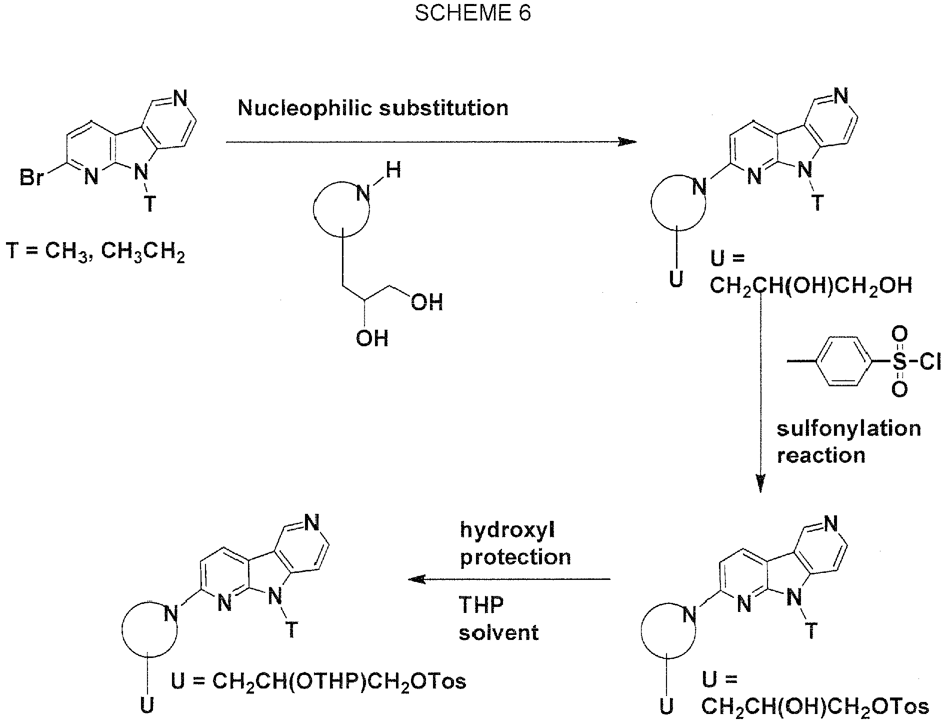

Tricyclic building blocks II and III were treated with cycloamine/bicycloamine derivatives containing a primary and a secondary hydroxyl-moiety in a solvent using a microwave to enable a nucleophilic substitution reaction to afford the desired bis-diol derivatives after purification. The primary hydroxyl group of derivatives with U = CH₂CH(OH)CH₂OH were treated with a suitable sulfonylation reagent (p-Tos-Cl) in a solvent (pyridine) to afford the desired derivatives with a tosyl-group at the primary alcohol. The secondary hydroxyl moiety is then treated with 3,4-dihydro-2H-pyran to afford the desired precursor compounds containing an acid labile protecting group at the secondary alcohol to allow the introduction of the ¹⁸F-label with subsequent cleavage of the THP-group in the following step.

### General synthesis of ¹⁸F-labeled compounds of the present invention

Compounds having the formula (I) which are labeled by ¹⁸F can be prepared by reacting a compound of formula (II) with an ¹⁸F-fluorinating agent, so that the leaving group LG is replaced by ¹⁸F. The preparation optionally includes the cleavage of a protecting group.

Any suitable ¹⁸F-fluorinating agent can be employed. Typical examples include H¹⁸F, alkali or alkaline earth ¹⁸F-fluorides (e.g., K¹⁸F, Rb¹⁸F, Cs¹⁸F, and Na¹⁸F). Optionally, the ¹⁸F-fluorination agent can be used in combination with a chelating agent such as a cryptand (e.g.: 4,7,13,16,21,24-hexaoxa-1,10-diazabicyclo[8.8.8]-hexacosane - Kryptofix^{®}) or a crown ether (e.g.: 18-crown-6). Alternatively, the ¹⁸F-fluorinating agent can be a tetraalkylammonium salt of ¹⁸F or a tetraalkylphosphonium salt of ¹⁸F. Examples thereof include tetrabutylammonium [¹⁸F]fluoride and tetrabutylphosphonium [¹⁸F]fluoride. Preferably, the ¹⁸F-fluorination agent is Cs¹⁸F, K¹⁸F, or tetrabutylammonium [¹⁸F]fluoride.

The reagents, solvents and conditions which can be used for the ¹⁸F-fluorination are well-known to a skilled person in the field (L. Cai, S. Lu, V. Pike, Eur. J. Org. Chem 2008, 2853-2873; J. Fluorine Chem., 27 (1985):177-191; Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). Preferably, the solvents used in the ¹⁸F-fluorination are DMF, DMSO, acetonitrile, DMA, or mixtures thereof, preferably the solvent is acetonitrile, DMSO.

If desired, the compound having the formula (II) can contain an amine protecting group in order to protect the group Z during the ¹⁸F fluorination reaction. This amine protecting group can be subsequently removed. Methods for removing the amine protecting group are known in the art and include, but are not limited to, acid cleavage.

If desired, the compound of formula (I) can be isolated and/or purified further before use. Corresponding procedures are well-known in the art.

The precursor compounds (II) of the present invention can be provided in a kit which is suitable for producing the compounds of the formula (I) by reaction with a ¹⁸F-fluorinating agent. In one embodiment the kit comprises a sealed vial containing a predetermined quantity of the precursor compound (II) of the present invention. For instance, the kit can contain 1.5 to 75 µmol, preferably 7.5 to 50 µmol, more preferably 10 to 30 µmol of a precursor compound (II) of the present invention. Optionally, the kit can contain further components, such as solvents, solid-phase extraction cartridges, components of the ¹⁸F-fluorinating agent, reagent for cleaving protecting groups, solvent or solvent mixtures for purification, solvents and excipients for formulation.

### Examples

All reagents and solvents were obtained from commercial sources and used without further purification. Proton (¹H) spectra were recorded on a Bruker DRX-400 MHz NMR spectrometer or on a Bruker AV-400 MHz NMR spectrometer in deuterated solvents. Mass spectra (MS) were recorded on an Advion CMS mass spectrometer. Chromatography was performed using silica gel (Fluka: Silica gel 60, 0.063-0.2 mm) and suitable solvents as indicated in the specific examples. Flash purification was conducted with a Biotage Isolera One flash purification system using HP-Sil or KP-NH SNAP cartridges (Biotage) and the solvent gradient indicated in the specific examples. Thin layer chromatography (TLC) was carried out on silica gel plates with UV detection.

Although some of the present examples do not indicate that the respective compounds were detectably labeled, it is understood that corresponding detectably labeled compounds are intended and can be easily prepared, e.g., by using detectably labeled starting materials, such as starting materials containing C(³H)₃, (¹¹C)H₃ or ¹⁸F.

### Preparative Example 1

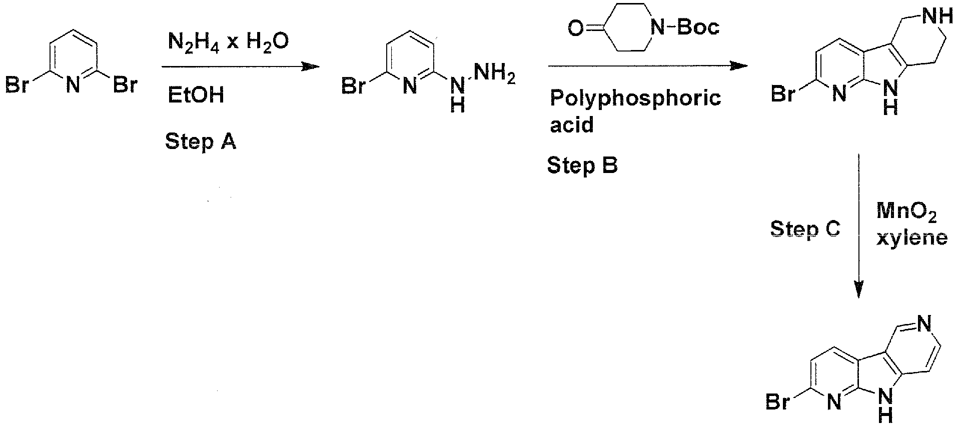

### Step A

Commercially available 2,6-dibromopyridine (4.12 g, 16.6 mmol) was suspended in ethanol (40 mL) and hydrazine hydrate (10 mL, 97.6 mmol) in water (∼50-60 %) was added. The mixture was heated in a sand-bath at ∼115 °C for 18 hours. The solvent was removed and the residue was purified by chromatography on silica using ethyl acetate/n-heptane (60/40) to afford the title compound as an off-white solid (3.05 g, 93 %).

¹H-NMR (400 MHz, CDCl₃): δ = 7.33 (t, 1 H), 6.83 (d, 1 H), 6.67 (d, 1 H), 6.00 (br-s, 1 H), 3.33-3.00 (br-s, 2H)

### Step B

The title compound from Step A above (10 g, 53.2 mmol) and commercially available 1-Boc-4-piperidone (10.6 g, 53.2 mmol) were added to a 500 mL flask and mixed to become a homogenous blend. Then polyphosphoric acid (80 g, 115% H₃PO₄ basis) was added and the mixture was heated at ∼160°C in a sand-bath. At ∼120°C the Boc-protecting group was cleaved resulting in foaming of the reaction mixture. After complete Boc-cleavage the foam collapsed and the dark reaction mixture was stirred at ∼160°C for 20 hours. The reaction was allowed to cool to room temperature and water (400 mL) was added. The reaction mixture was stirred/sonicated until the gummy material was dissolved. The reaction mixture was then placed in an ice-bath and the pH of the solution was adjusted to pH ∼12 by adding solid sodium hydroxide pellets (exothermic!). The precipitate was collected by filtration and washed with water (400 mL) to remove salts. The precipitate was dissolved in dichloromethane/methanol (9/1; 1500 mL) by sonication and washed with water (2 x 400 mL) to remove remaining salts and insoluble material. The organic phase was dried over Na₂SO₄, filtered and the solvents removed under reduced pressure. The dark residue was treated with dichloromethane (100 mL), sonicated for 5 minutes and the precipitate collected by filtration. The precipitate was washed with dichloromethane (40 mL) and air-dried to afford the title compound a beige solid (3.5 g, 26 %).

¹H-NMR (400 MHz, DMSO-d₆): δ = 11.5 (br-s, 1H), 7.72 (d, 1H), 7.15 (d, 1H), 3.86-3.82 (m, 2H), 3.06-3.00 (m, 2H), 2.71-2.65 (m, 2H)

### Step C

The title compound from Step B above (1.75 g, 6.94 mmol) was suspended in xylene (380 mL) and manganese (IV) oxide (6.62 g, 76.9 mmol) was added. The reaction mixture was then heated at ∼160°C in a sand-bath for 36 hours. The cooled reaction mixture was evaporated under reduced pressure, the residue suspended in dichloromethane/methanol (1/1; 400 mL) and stirred at room temperature for 30 minutes. The reaction mixture was then filtered through paper filters to remove the manganese (IV) oxide and the filter washed with methanol (50 mL). The combined filtrates were evaporated under reduced pressure and the dark residue purified by chromatography on silica (50 g HP-SIL-cartridge) using a Biotage Isolera system employing an ethyl acetate/heptane gradient (5/95-100/0) to remove unpolar impurities followed by dichloromethane/methanol (9/1 -> 4/1) to afford the title compound as dark yellow solid. The total yield from 2 runs was 1.77 g (51 %).

¹H-NMR (400 MHz, DMSO-d₆). δ = 12.52 (br-s, 1H), 9.42 (s, 1H), 8.61 (d, 1H), 8.53 (d, 1H), 7.56-7.52 (m, 2H)

### Preparative Example 2

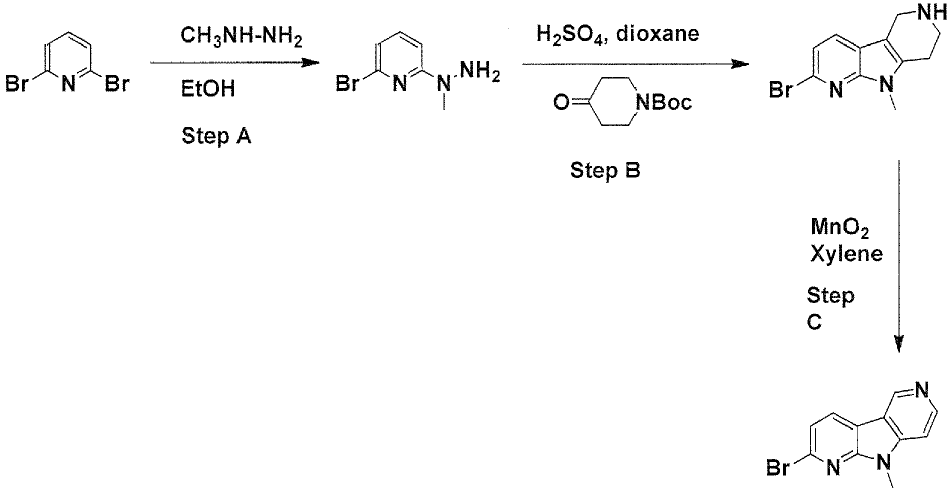

### Step A

To a suspension of commercially available 2,6-dibromopyridine (10 g, 42.2 mmol) in ethanol (50 mL) was added commercially available *N*-methylhydrazine (11.11 mL, 211 mmol). The mixture was heated at 80 °C (reaction mixture temperature) for 48 h. The reaction mixture was concentrated to dryness and the residue was purified by chromatography on HP-Sil SNAP cartridges using a Biotage Isolera One purification system employing an ethyl acetate/n-heptane gradient (15/75 -> 35/65) to afford the title compound as a reddish oil which became a solid by standing at room temperature (7.6 g, 89 %)

¹H-NMR (400 MHz, CDCl₃): δ = 3.23 (s, 3H), 4.00 (br-s, 2H), 6.70 (d, 1H), 6.82 (d, 1H), 7.27 (t, 1 H)

### Step B

A suspension of *N*-Boc-piperidone (10 g, 56 mmol) and the title compound from Step A above (10.1 g, 50 mmol) in 1,4-dioxane (100 mL) was placed in an ice-bath. To the stirred suspension concentrated sulfuric acid (15 mL) was slowly added (exothermic, gas evolution). After the addition was completed, the reaction mixture was heated at reflux temperature for 7 h using a sand-bath (∼140 °C). The reaction mixture was cooled to room temperature, the dioxane layer was discarded, and ice-water (150 mL) was added. The mixture was stirred until the gummy material was dissolved. Then the pH of the reaction mixture was adjusted to pH = 12-13 by adding solid NaOH. After adjusting the pH, the reaction mixture was stirred at 0°C for 30 minutes, the precipitate was collected by filtration and washed with water (15 mL). The precipitate was suspended in propan-2-ol (80 mL) and heated at reflux for 10 minutes. The reaction mixture was filtered, the precipitate was washed with propan-2-ol (10 mL) and the combined filtrate was evaporated. The dark residue was purified by chromatography on a 100 g KP-NH column using a Biotage Isolera One purification system employing a dichloromethane/methanol gradient (100/0 -> 80/20) to afford the title compound as a grey solid (3.14 g, 23 %).

¹H-NMR (400 MHz, CDCl₃): δ = 2.74-2.78 (m, 2H), 3.26 (t, 2H), 3.70 (s, 3H), 4.02 (t, 2H), 7.13 (d, 1 H), 7.52 (d, 1 H)

### Step C

The title compound from Step B above (3.0 g, 11.28 mmol) was dissolved in xylene (230 mL) and activated MnO₂ (10.8 g, 124 mmol) was added at room temperature. The mixture was then heated at ∼160°C in a sand-bath for 24 hours. The reaction mixture was cooled to room temperature, filtered through paper filters and the filter was washed with xylene (40 mL). The combined filtrates were evaporated under reduced pressure and the dark residue was purified by chromatography on HP-Sil SNAP cartridges using a Biotage Isolera One purification system employing an EtOAc/n-heptane gradient (5/95 -> 100/0) to elute nonpolar by-products. Then the gradient was changed to dichloromethane/methanol (90/10) to afford the title compound as a dark-yellow solid (2.0 g, 64 %).

¹H-NMR (400 MHz, DMSO-d₆): δ = 3.90 (s, 3H), 7.57 (d, 1H), 7.74 (d, 1H), 8.60-8.63 (m, 2H), 9.42 (s, 1 H)

### Preparative Example 3

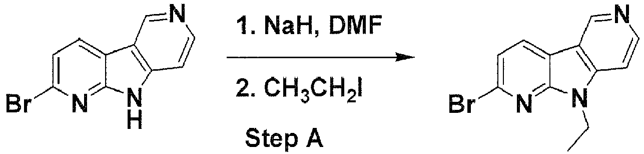

### Step A

To a suspension of the title compound from Preparative Example 1 (0.1 g, 0.403 mmol) in *N,N*'-dimethylformamide (5 mL) at 0°C was added sodium hydride 95% (0.0122 mg, 0.484 mmol). After 1 hour at 0°C (a clear solution was obtained), iodoethane (0.039 mL, 0.484 mmol) was slowly added and the stirring continued for 6 hours at room temperature. Saturated aqueous solution of ammonium chloride (50 mL) followed by dichloromethane (75 mL) were added to the reaction mixture. The organic phase was then washed with water (3 x 50 mL), dried over sodium sulfate, filtered and dried under reduced pressure to afford the title compound (0.096 mg, 86 %).

¹H-NMR (400 MHz, DMSO-d₆) δ 9.43 (s, 1 H), 8.61 (t, 2H), 7.78 (d, 1 H), 7.57 (d, 1 H), 4.48 (q, 2H), 1.37 (t, 3H).

MS (ESI); m/z = 276.37 [M+H]+

### Preparative Example 4

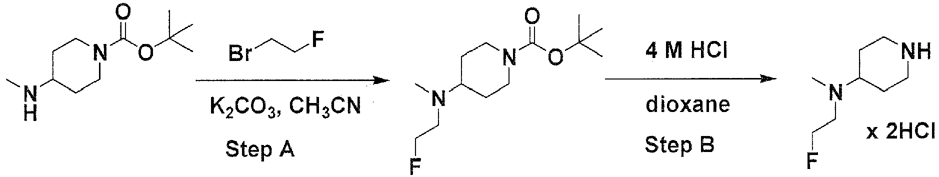

### Step A

To a solution of commercially available tert-butyl 4-(methylamino)piperidine-1-carboxylate (1.0 g, 4.6 mmol) in acetonitrile (40 mL), K₂CO₃ (1.9 g, 14.0 mmol) was added and the mixture was stirred at room temperature for 30 minutes. Then 1-bromo-2-fluoroethane (0.827 g, 6.9 mmol) was added dropwise. The mixture was heated at 65 °C overnight. To the cooled reaction mixture H₂O (20 ml) was added and the product was extracted with ethyl acetate (3 x 50 ml), the organic layers were combined, dried over Na₂SO₄ and evaporated under reduced pressure. The residue was purified on HP-Sil SNAP cartridges using a Biotage Isolera One purification system employing an EtOAc/n-heptane gradient (5/95 -> 40/60) to afford the title compound (0.539 g, 45 %).

¹H-NMR (400 MHz, DMSO-d₆) δ = 4.46 (dt, 2H), 3.97 (d, 2H), 2.70 (dt, 4H), 2.52 (p, 1 H), 2.22 (s, 3H), 1.66 (dq, 2H), 1.40 (s, 9H), 1.25 (qd, 2H)

### Step B

Solutions of the title compound from Step A above (0.539 g, 2.07 mmol) in 4 N solution of HCl in dioxane (5 mL) and dioxane (10 mL) was stirred at room temperature for 18 hours. The white solid was filtered and washed with diethyl ether (10 mL) and dried *in vacuo* to afford the title compound as a HCl salt (0.278 g, 58 %).

¹H-NMR (400 MHz, DMSO-d₆) δ = 11.52 (s, 1H), 9.25 (d, 2H), 5.13-4.67 (m, 2H), 3.66-3.48 (m, 3H), 3.03-2.83 (m, 2H), 2.75 (d, 3H), 2.23 (dd, 2H), 2.00 (tq, 2H).

MS (ESI); m/z= 161.31 [M+H]⁺

### Example 1

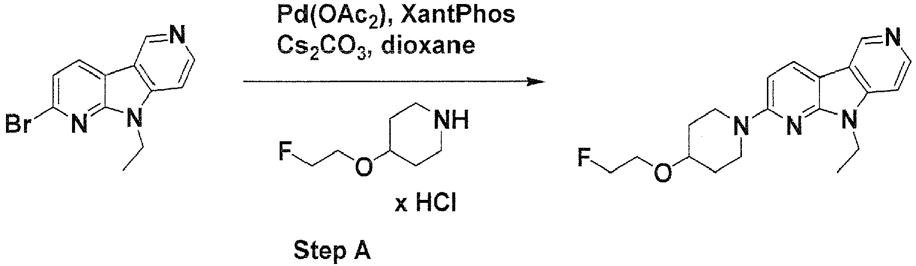

### Step A

1,4-Dioxane (4 mL) was degassed for 10 minutes and palladium(II)-acetate (0.002 g, 0.0095 mmol) and 5-bis(diphenylphosphino)-9,9-dimethylxanthene (0.0157 g, 0.027 mmol) were added. The suspension was then heated at 110°C for 2 minutes. Then the title compound from Preparative Example 3 (0.025 g, 0.091 mmol), commercially available 4-(2-fluoroethoxy)piperidine hydrochloride (0.022 g, 0.118 mmol) and cesium carbonate (0.118 g, 0.362 mmol) were added and heating was continued at 110°C for 2 hours. The reaction mixture was cooled to room temperature and 1 N NaOH (20 mL) was added. The aqueous phase was then extracted with dichloromethane (3 x 30 mL). The combined organic phase were dried over sodium sulfate, filtered and the solvents removed under reduced pressure. The crude was purified on HP-Sil SNAP cartridges using a Biotage Isolera One purification system employing a methanol/dichloromethane gradient (2/98 -> 10/90) to afford the title compound (0.016 g, 52 %).

¹H-NMR (400 MHz, DMSO-d₆) δ = 9.11 (s, 1 H), 8.37 (d, 1 H), 8.30 (d, 1 H), 7.56 (d, 1 H), 6.86 (d, 1H), 4.54 (dt, 2H), 4.39 (q, 2H), 4.15 (dt, 2H), 3.80-3.59 (m, 3H), 3.32-3.24 (m, 2H), 2.05-1.89 (m, 2H), 1.51 (dtd, 2H), 1.34 (t, 3H).

MS (ESI); m/z = 343.48 [M+H]+

### Example 2

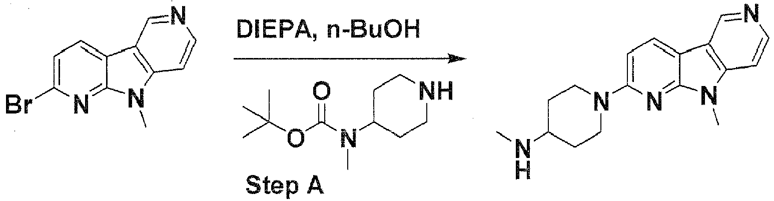

### Step A

To a microwave tube were added the title compound from Preparative Example 2 (0.1 g, 0.19 mmol), tert-butyl methyl(piperidin-4-yl)carbamate (0.326 g, 0.76 mmol), n-butanol (4 mL), followed by *N,N*'-diisopropyl ethylamine (0.460 mL, 1.33 mmol). The tube was sealed and heated at 200 °C for 3 hours using a Biotage Initiator microwave. The solvents were removed under reduced pressure and NH₄Cl saturated solution (25 ml) was added to the mixture followed by DCM (20 ml). The phases were separated and the aqueous layer was extracted with dichloromethane (2 x 20 ml). The organics were collected and dried over Na₂SO₄. The solvent was removed under reduced pressure and the residue was purified on HP-Sil SNAP cartridges using a Biotage Isolera One purification system employing a MeOH/DCM gradient (0/100 -> 10/90) to afford the title compound (0.055 g, 98 %).

¹H-NMR (400 MHz, DMSO-d₆) δ = 9.09 (d, 1 H), 8.37 (d, 1 H), 8.27 (d, 1 H), 7.50 (dd, 1H), 6.82 (d, 1H), 4.35 (dt, 2H), 3.79 (s, 3H), 3.07 (m, 2H), 2.32 (s, 3H), 2.01-1.85 (m, 3H), 1.32-1.20 (m, 3H).

MS (ESI); m/z = 296.48 [M+H]⁺

### Examples 3 to 11

Following the coupling procedure as described in Example 2, except using the bromo and amino or amino-fluorine derivatives indicated in the table below, the following compounds were prepared. In case the HCl-salt of an amine was used, one additional equivalent of diisopropyl ethylamine was added.

**Table 1:**

| Bromo derivative | Amino-derivative/ Amino-fluorine derivative | Product | 1. Yield |
|---|---|---|---|
| | | **Example** | 2. ¹H-NMR |
| | | | 3. MH⁺ (ESI) |
| 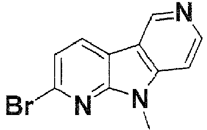 | 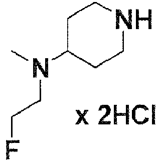 | 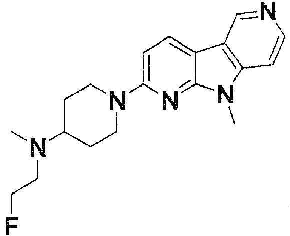 | 1.27% |
| | | | 2. ¹H-NMR (400 MHz, CDCl₃) δ = 9.08 (s, 1H), 8.48 (d, 1H), 8.13 (d, 1H), 7.28 (d, 1H), 6.67 (d, 1H) 4.62 (dd, 3H), 4.50 (t, 1H), 3.86 (s, 3H), 2.95 (td, 2H), 2.87 (t, 1H), 2.84-2.72 (m, 2H), 2.56 (s, 0H), 2.40 (s, 3H), 2.00-1.88 (m, 2H), 1.63 (qd, 2H). |
| | | **3** | 3. 342.41 |
| 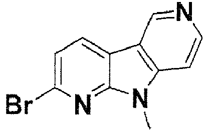 | 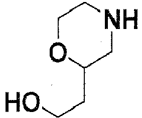 | 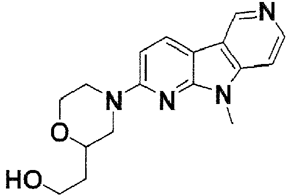 | 1.47% |
| | | | 2. ¹H-NMR (400 MHz, CDCl₃) δ = 9.05 (d, 1H), 8.65-8.37 (m, 1H), 8.06 (d, 1H), 7.32-7.10 (m, 1H), 6.70-6.39 (m, 1H), 4.36-4.16 (m, 2H), 4.15-4.05 (m, 1H), 3.96-3.87 (m, 3H), 3.88-3.65 (m, 5H), 3.08 (td, 1H), 2.90-2.65 (m, 1H), 1.90 (qq, 2H) |
| | | **4** | |
| 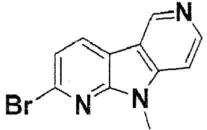 | 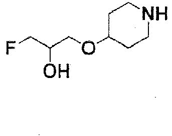 | 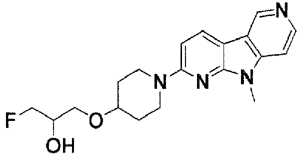 | 1.32% |
| | | | 2. ¹H-NMR (400 MHz, CDCl₃) δ = 9.09 (s, 1H), 8.48 (d, 1H), 8.14 (d, 1H), 7.28 (s, 2H), 6.67 (d, 1H), 4.71-4.36 (m, 2H), 4.29-3.99 (m, 3H), 3.86 (s, 3H), 3.75-3.58 (m, 3H), 3.52-3.30 (m, 2H), 2.14-1.95 (m, 2H), 1.86-1.59 (m, 2H) |
| | | **5** | |
| 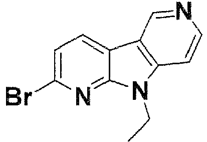 | 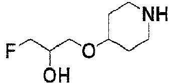 | 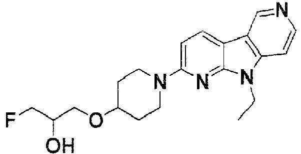 | 1.35% |
| | | | 2. ¹H-NMR (400 MHz, CDCl₃) δ = 9.09 (s, 1H), 8.46 (s, 1H), 8.14 (d, 1H), 7.35 (d, 1H), 6.71 (d, 1H), 4.63-4.50 (m, 1H), 4.53-4.34 (m, 3H), 4.22-3.95 (m, 4H), 3.77-3.56 (m, 3H), 3.53-3.34 (m, 2H), 2.14-1.94(m, 2H), 1.92-1.60 (m, 2H), 1.48 1.60 (m, 2H), 1.48 (t, 3H) |
| | | **6** | |
| 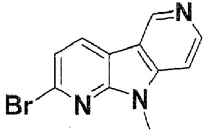 | 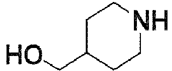 | 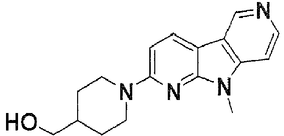 | 1.75% |
| | | | 2. ¹H-NMR (400 MHz, CDCl₃) δ = 9.06 (s, 1H), 8.46 (d, 1H), 8.11 (d, 1H), 7.29 (s, 1H), 6.67 (d, 1H), 4.58 (dt, 2H), 3.86 (s, 3H), 3.59 (d, 2H), 2.99 (td, 2H), 2.35 (s, 1H), 2.02-.79 (m, 3H), 1.51-1.19 (m, 2H) |
| | | **7** | 3. 297.46 |
| 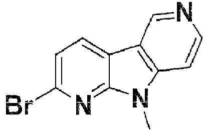 | 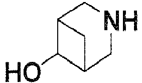 | 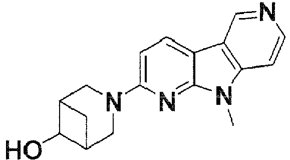 | 1.48% |
| | | | 2. ¹H-NMR (400 MHz, CDCl₃) δ = 8.72 (s, 1H), 8.37 (d, 1H), 7.85 (d, 1H), 7.11 (d, 1H), 6.38 (d, 1H), 4.26 (t, 1H), 3.81 (q, 4H), 3.64 (s, 3H), 2.81 (t, 2H), 1.73 (dt, 1H), 1.47 (d, 1H) |
| | | **8** | 3. 295.38 |
| 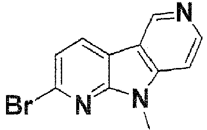 | 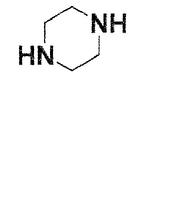 | 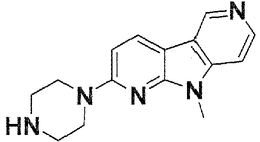 | 1. |
| | | | 2. ¹H-NMR (400 MHz, DMSO-d₆) δ = 9.09 (s, 1H), 8.37 (d, 1H), 8.29 (d, 1H), 7.50 (d, 1H), 6.78 (d, 1H), 3.78 (s, 3H), 3.65-3.53 (m, 4H), 2.87-2.76 (m, 4H) |
| | | **9** | 3. 268.17 |
| 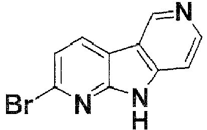 | 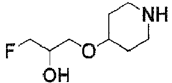 | 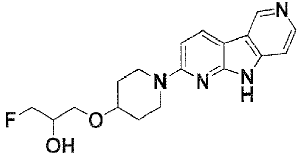 | 1. 17 % |
| | | | 2. ¹H-NMR (400 MHz, DMSO-d₆) δ 11.74 (s, 1H), 9.08 (s, 1H), 8.48-8.16 (m, 2H), 7.30 (d, 1H), 6.82 (d, 1H), 5.15 (d, 1H), 4.62-4.19 (m, 2H), 4.14-3.92 (m, 2H), 3.92-3.68 (m, 1H), 3.71-3.52 (m, 1H), 3.50-3.42 (m, 2H), 2.01-1.81 (m, 2H), 1.56-1.28 (m, 2H) |
| | | **10** | 3. 345.25 |
| 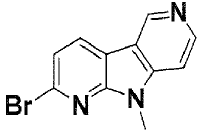 | 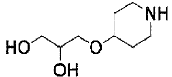 | 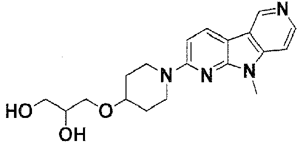 | 1.50% |
| | | | 2. ¹H-NMR (400 MHz, CDCl₃) 5 = 9.03 (d, 1H), 8.44 (d, 1H), 8.06 (d, 1H), 7.24 (dd, 1H), 6.59 (d, 1H), 4.17-4.04 (m, 2H), 3.98-3.87 (m, 1H), 3.83-3.75 (m, 4H), 3.74-3.54 (m, 4H), 3.44-3.30 (m, 2H), 2.13-1.91 (m, 2H), 1.82-1.59 (m, 2H). |
| | | **11** | |

### Example 12

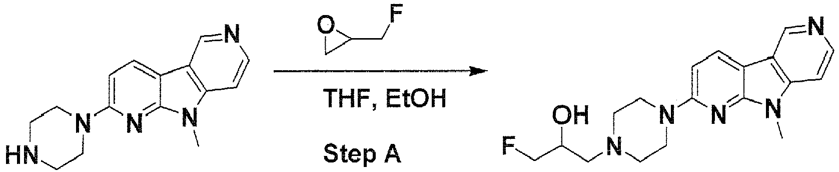

### Step A

A solution of the title compound from Example 9 (0.0216 mg, 0.081 mmol) and 2-(fluoromethyl)oxirane (0.00738 g, 0.097 mmol) in tetrahydrofurane (1 mL) and ethanol (1 mL) was heated at 170°C for 20 minutes under microwave irradiation. The crude was concentrated under reduced pressure and purified on HP-Sil SNAP cartridges using a Biotage Isolera One purification system employing a methanol/dichloromethane gradient (2/98 -> 15/85) to afford the title compound (0.006 g, 22 %).

¹H-NMR (400 MHz, DMSO-d₆) δ = 9.11 (s, 1 H), 8.38 (d, 1 H), 8.31 (d, 1 H), 7.52 (d, 1 H), 6.81 (d, 1H), 5.00 (d, 1H), 4.55-4.24 (m, 2H), 3.99-3.84 (m, 1H), 3.80 (s, 3H), 3.66 (t, 4H), 2.58 (qt, 4H), 2.44 (dd, 1 H), 2.40-2.33 (m, 1 H).

MS (ESI); m/z = 344.14 [M

### Example 13

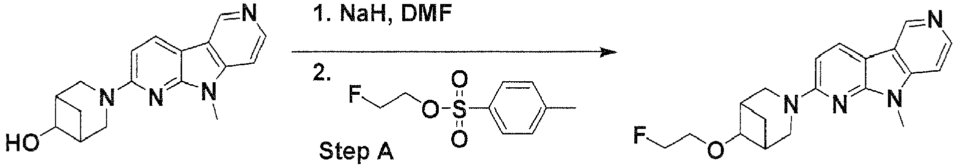

### Step A

To a solution of of the title compound from Example 8 (0.025 g, 0.085 mmol) in *N,N'-*dimethylformamide (5 mL) was added sodium hydride (0.006 g 0.255 mmol). The suspension was stirred at room temperature for 10 minutes and heated at 70°C for 1 hour. The mixture was cooled at room temperature and 2-fluoroethyl 4-methylbenzenesulfonate (0.022 g, 0.102 mmol) was added. The reaction mixture was heated at 70°C overnight. Then 0.1 mL of water was added and the solvent were removed under reduced pressure and the residue was purified on HP-Sil SNAP cartridges using a Biotage Isolera One purification system employing a dichloromethane/methanol gradient (100/0 -> 90/10) to afford the title compound (0.011 g, 38 %).

¹H-NMR (400 MHz, CDCl₃) δ = 9.07 (s, 1 H), 8.45 (d, 1 H), 8.12 (d, 1 H), 7.29 (d, 1 H), 6.52 (d, 1H), 4.53 (ddd, 2H), 3.96-3.84 (m, 4H), 3.83-3.79 (m, 1 H), 3.79-3.69 (m, 3H), 3.01 (m, 2H), 2.89-2.82 (m, 2H), 1.72 (dt, 1 H), 1.56 (d, 1 H).

MS (ESI); m/z = 340.88 [M+H]⁺

### Example 14

Following the alklyation procedure as described in Example 13, except using the alcohol derivatives and alkylation reagents indicated in the table below, the following compounds were prepared.

**Table 2:**

| Alcohol derivative | Alkylation reagent | Product | 1. Yield |
|---|---|---|---|
| | | **Example** | 2. ¹H-NMR |
| | | | 3. MH⁺ (ESI) |
| 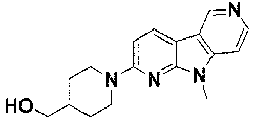 | 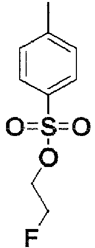 | 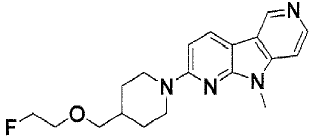 | 1.7% |
| | | | 2. ¹H-NMR (400 MHz, CDCl₃) δ = 9.08 (s, 1H), 8.47 (s, 1H), 8.11 (d, 1H), 7.32 (d, 1H), 6.68 (d, 1H), 4.68-4.47 (m, 4H), 3.87 (s, 3H), 3.72 (dt, 2H), 3.42 (d, 2H), 2.99 (td, 2H), 2.05-1.82 (m, 3H), 1.51-1.09 (m, 2H) |
| | | **14** | 3. 343.53 |

### Example 15

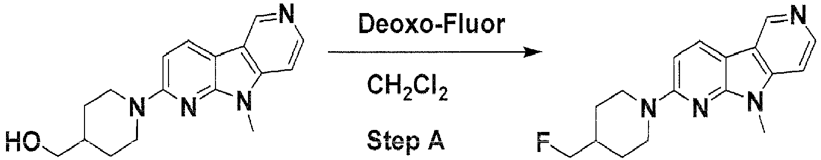

### Step A

To a solution of the title compound from Example 7 (0.06 g, 0.207 mmol) in dichloromethane (4 mL) at 0°C was added a 50 % solution of bis(2-methoxyethyl)aminosulfuric trifluoride (Deoxo-Fluor^{®}) in terahydrofurane (0.358 mL, 0.81 mmol). The reaction mixture was then stirred at room temperature for 4 hours. The reaction mixture was poured into water (5 mL) and the aqueous phase was extracted with dichloromethane several times. The combined organics were dried over Na₂SO₄, filtered and concentrated *in vacuo*. The residue was purified on Redisep^{®} gold silica cartridges using a Biotage Isolera One purification system employing a dichloromethane/methanol gradient (100/0 -> 90/10) to afford the title compound (0.011 g, 17 %).

¹H-NMR (400 MHz, CDCl₃) δ = 9.08 (s, 1H), 8.46 (d, 1H), 8.13 (d, 1H), 7.36 (d, 1H), 6.70 (d, 1 H), 4.61 (dt, 2H), 4.35 (dd, 2H), 3.88 (s, 3H), 3.02 (td, 2H), 2.05 (m, 1H), 1.90 (dd, 2H), 1.44 (qd, 2H).

MS (ESI); m/z = 299.38 [M+H]⁺

### Examples 16 to 17

Following the fluorination procedure as described in Example 15, except using the alcohol derivatives indicated in the table below, the following compounds were prepared.

**Table 3:**

| Alcohol derivative | Product | 1. Yield |
|---|---|---|
| | **Example** | 2. ¹H-NMR |
| | | 3. MH⁺ (ESI) |
| 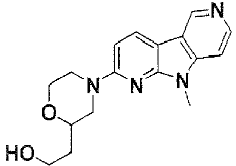 | 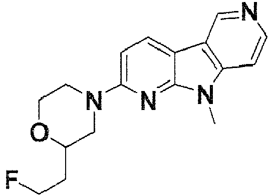 | 1. 16 % |
| | | 2. ¹H-NMR (400 MHz, CDCl₃) δ = 9.11 (s, 1H), 8.50 (d, 1H), 8.18 (dd, 1H), 7.34 (dd, 1H), 6.66 (dd, 1H), 4.84-4.53 (m, 2H), 4.41-4.30 (m, 1H), 4.30-4.19 (m, 1H), 4.16-4.03 (m, 1H), 3.89(s, 3H), 3.85-3.70 (m, 2H), 3.20-3.04 (m, 1H), 2.90-2.76 (m, 1H), 2.19-1.86 (m, 2H) |
| | **16** | |
| 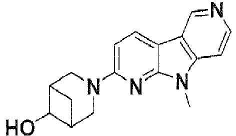 | 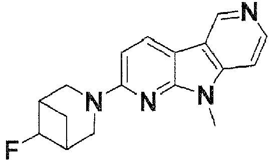 | 1.32% |
| | | 2. ¹H-NMR (400 MHz, CDCl₃) δ = 9.07 (s, 1H), 8.47 (d, 1H), 8.12 (m, 1H), 7.37-7.18 (m, 1H), 6.55 (m, 1H), 5.45-5.14 (m, 1H), 4.31-4.20 (m, 1H), 4.02 (m, 1H), 3.85 (m, 4H), 3.75-3.42 (m, 1H), 3.14 (s, 2H), 1.64-1.41 (m, 1H), 0.97-0.46 (m, 1H). |
| | **17** | |
| | | 3. 297.53 |

### Example 18

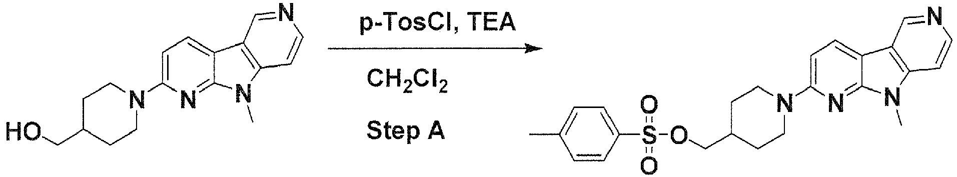

### Step-A

To a solution of the title compound from Example 7 (0.05 g, 0.146 mmol) in dichloromethane (5 mL) triethylamine (0.028 mL, 0.202 mmol) was added and the mixture was cooled at 0 °C. Then tosylsulfonyl chloride (0.038 mg, 0.202 mmol) was added and the mixture was stirred at 0 °C for 30 minutes and at room temperature for 2 hours. Another equivalent of triethylamine, followed by another equivalent of tosylsulfonyl chloride was added to the reaction mixture at room temperature and stirred for 2 hours at the some temperature. Another equivalent of triethylamine, followed by another equivalent of tosylsulfonyl chloride was added to the reaction mixture at room temperature in order to get complete conversion of the starting material. A saturated ammonium chloride solution (20 mL) was added to the mixture, followed by dichloromethane (10 mL). The phases were separated and the organic layer was washed with saturated ammonium chloride solution (20 mL) and water (20 mL). The organic phase was dried over Na₂SO₄ and the solvent was removed under reduced pressure. The residue was purified on HP-Sil SNAP cartridges using a Biotage Isolera One purification system employing a dichloromethane/methanol gradient (100/0 -> 90/10) and the crude was triturated with diisopropyl ether (10 mL) and the solid was collected to afford the title compound (0.055 g, 73 %).

¹H-NMR (400 MHz, DMSO-d₆) 5 = 9.10 (s, 1 H), 8.38 (d, 1H), 8.28 (d, 1H), 7.80 (d, 2H), 7.51 (d, 1H), 7.12 (d, 2H), 6.81 (d, 1H), 4.49 (d, 2H), 3.94 (d, 2H), 3.79 (s, 3H), 2.89 (td, 2H), 2.42 (s, 3H), 2.06-1.83 (m, 1 H), 1.77-1.62 (m, 2H), 1.30-1.11 (m, 2H)

MS (ESI); m/z = 451.56 [M+H]⁺

### Examples 19 to 20

Following the alklyation procedure as described in Example 18, except using the alcohol derivatives and alkylation reagents indicated in the table below, the following compounds were prepared.

**Table 4:**

| Alcohol derivative | Alkylation reagent | Product | 1. Yield |
|---|---|---|---|
| | | **Example** | 2. ¹H-NMR |
| | | | 3. MH⁺ (ESI) |
| 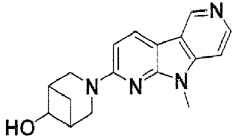 | 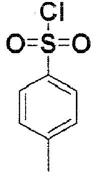 | 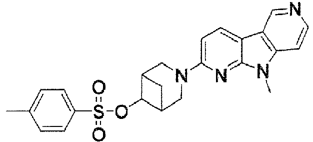 | 1.24% |
| | | | 2. ¹H-NMR (400 MHz, DMSO-d₆) δ = 9.15 (s, 1H), 8.41 (d, 1H), 8.35 (d, 1H), 7.90-7.80 (m, 2H), 7.59 (d, 1H), 7.48 (d, 2H), 6.59 (d, 1H), 4.82 (t, 1H), 3.83 (s, 3H), 3.55 (d, 2H), 2.79 (t, 2H), 2.43 (s, 3H), 1.79 (dt, 1H), 1.50 (d, 1H), 1.25 (m, 2H). |
| | | **19** | 3. 449.49 |
| 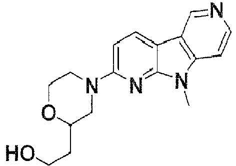 | 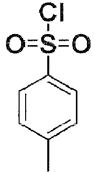 | 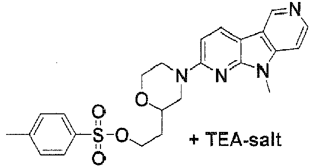 | 1.65% |
| | | **20** | |

### Example 21

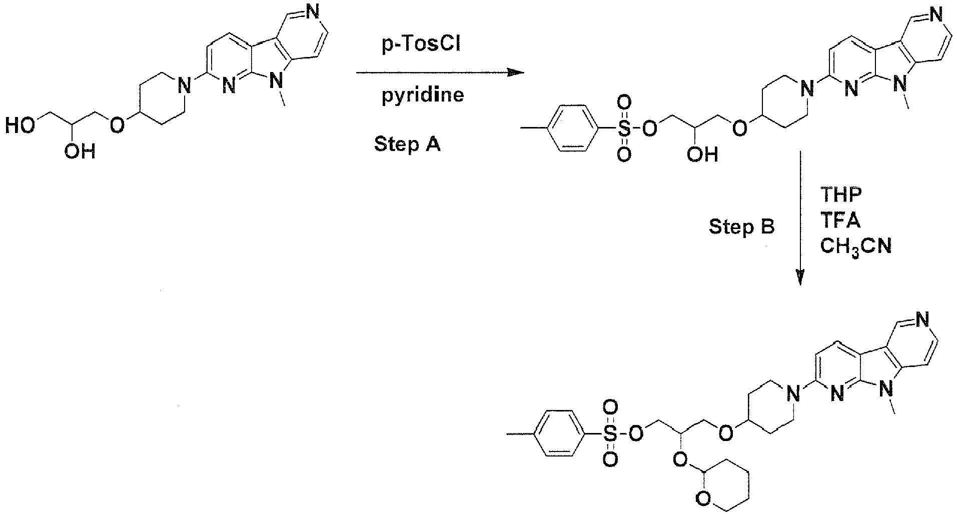

### Step A

To a solution of the title compound from Example 11 (0.125 g, 0.35 mmol) in tetrahydrofuran (15 mL) was added pyridine (0.100 mL, 1.05 mmol) and reaction mixture was cooled to 0°C. Then, toluene sulfonyl chloride (0.1 g, 0.52 mmol) was added, the reaction mixture was stirred at 0°C for 2 hours and brought to room temperature over a period of 1 hour. Then the reaction mixture was diluted with dichloromethane (100 mL). The organic phase was washed with water/brine and dried over Na₂SO₄. The solvent was removed and the crude was purified on HP-Sil SNAP cartridges using a Biotage Isolera One purification system employing a dichloromethane/methanol gradient (100/0 -> 80/20) to afford the title compound (0.14 g, 78 %).

MS (ESI); m/z = 511.42 [M+H]⁺

### Step B

To a solution of title compound from Step A above (0.05 g, 0.098 mmol) in acetonitrile ( 4 mL) was added 3,4-dihydro-2H-pyran (0.3 g, 3.57 mmol) and trifluoro acetic acid (0.0020 mL, catalytic amount). The reaction mixture was stirred at room temperature overnight. The solvent was removed, and the crude was re-dissolved in dichloromethane (100 mL), washed with water/brine and dried over Na₂SO₄. The solvent was removed to afford the title compound (0.025 g, 43 %).

MS (ESI); m/z = 595.65 [M+H]⁺

### Synthesis of ¹⁸F-labeled compounds

### General Method (direct ¹⁸F-labeling plus deprotection)

The tracers were synthesized starting from n.c.a. [¹⁸F]fluoride (1-10 GBq) by a ¹⁸F-direct fluorination. The aqueous [¹⁸F]fluoride solution was trapped on a Sep-Pak Accell Plus QMA light cartridge (Waters) and eluted with a solution K₂CO₃/Kryptofix^{®} 2.2.2. The water was removed using a stream of N₂ at 120 °C and co-evaporated to dryness with MeCN (3 x 1 mL). Afterwards, the respective dissolved precursor was added to the dried K[¹⁸F]F-K₂₂₂ complex. The reaction vial was sealed and heated for 15 min at 120-160 °C (heating block). For deprotection hydrochloric acid was added and stirred for another 10 min at 110 °C. After neutralization using sodium hydroxide solution the reaction mixture was quenched with ammonium formate buffer and trapped on a C-18 Plus cartridge (Waters). The cartridge was washed with water (5 mL), eluted with acetonitrile and subsequently, the crude product was purified via semi-preparative HPLC. The isolated tracer was diluted with water (25 mL), trapped on a C-18 Plus cartridge (Waters), washed with water (5 mL), eluted with ethanol (1 mL).

### Representative examples of ¹⁸F-radiolabeling:

### Example ¹⁸F-5

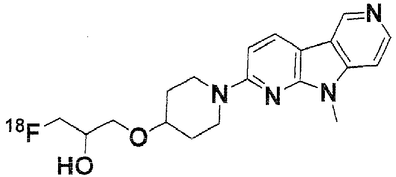

¹⁸F-5 (154 MBq) was synthesized according to General Method using precursor molecule from Example 21 (5 mg, 12 µmol) in dimethylformamide (0.5 mL).

The radiochemical purity of 100% was determined by analytical reversed-phase HPLC (t_{R}(RAD-trace)= 2.98 min). The identity of ¹⁸F-5 was confirmed by comparing the retention time with the non-radioactive reference from Example 5 .

### Biological Assay Description

### Assay 1 (Fluorescence based assay):

### Direct binding of compounds of this invention to human Alzheimer's disease brain sections

Frozen sections with a thickness of 20 µM from the amygdala of donors diagnosed with Braak stage V-VI Alzheimer's Disease (Braak, H.; Braak, E. Acta Neuropathol., 1991, 82, 239-259), were purchased from a commercial provider (Tissue Solutions) and kept at -80°C until use.

### Direct staining of human Alzheimer's disease brain sections

Brain sections were encircled with pap pen liquid blocker to reduce the volume of solution for the different incubations. Sections were fixed for 15 min at 4°C with 4% paraformaldehyde and washed three times 5 minutes with PBS at room temperature. Test compounds were incubated on the sections at 100 µM in 50% ethanol in water for 20 min at room temperature in the dark and then washed three times 5 minutes with PBS. To reduce the auto-fluorescence of the tissue, the sections were incubated in a solution of 0.1% Sudan Black (Sigma 199664) in 70% ethanol for 15 min at room temperature in the dark, washed four times for 5 minutes with PBS, and mounted using ProLong Gold Antifade reagent (Invitrogen P36930). Sections were then analyzed on the Nikon Eclipse Ti microscope to detect staining and imaged using Nikon DS-Fi2 camera and NIS-Element AR4.13.1 software.

**The following Example compounds were measured in Assay 1:**

**Table 5:**

| **Example** | **Structure** | **Direct staining of tau tangles/Aβ plaques on AD brain sections at 100 µM** |
|---|---|---|
| **1** | 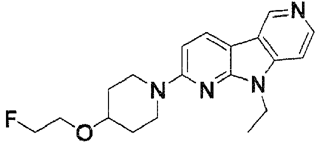 | - |
| **3** | 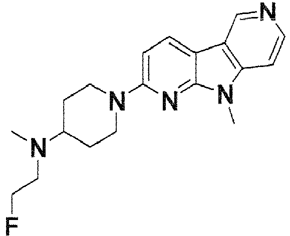 | - |
| **5** | 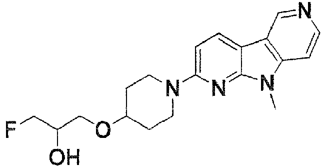 | ++ |
| | | **(tangles)** |
| **6** | 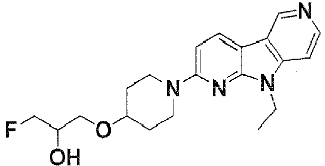 | ++ |
| | | **(tangles)** |
| **10** | 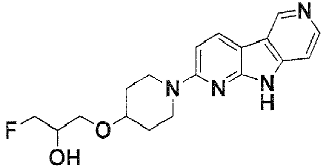 | **n.d.** |
| **12** | 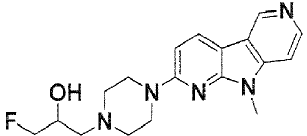 | + |
| | | **(tangles)** |
| **13** | 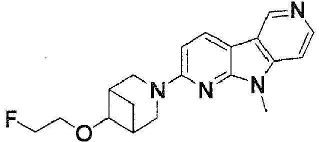 | ++ |
| | | **(tangles)** |
| **14** | 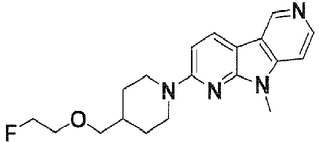 | - |
| **15** | 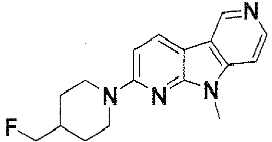 | **n.d.** |
| **16** | 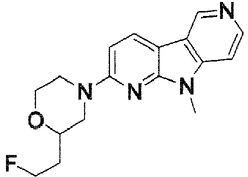 | - |
| **17** | 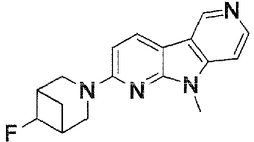 | **n.d.** |

| | | |
|---|---|---|
| Direct tau tangle/Aβ plaque staining: - (No); +++ (strong); ++ (good); + (weak); n.d.: not determined; | | |

### Tau radio-binding competition assay

20 µg of human Alzheimer diseased brain homogenate (stage Braack V) was incubated with a dilution series of each test compound (1000 to 0.06 nM) in the presence of 800 Bq of [¹⁸F]labeled Tau-Reference binder. The samples were shaked at 110 rpm for 45 min at 37°C. Samples were then filtered through GF/B 96well filter plates and two times washed with 300 µl assay buffer (PBS containing 0.1% BSA and 2% DMSO). Thereafter, filter plates were sealed and a Fuji Film Imaging Plate (BAS-SR2025) were placed on top. The imaging plate was analyzed the next day using a Fuji Film BAS-5000. Non-specific signal was determined with samples containing [¹⁸F]labeled Tau-Reference binder in the presence of assay buffer missing brain substrate and competitor. Specific binding was calculated by subtracting the non-specific signal from the measured sample signal. The unblocked

[¹⁸F]labeled Tau-Reference binder signal was defined as total binding. IC₅₀ values were calculated by Prism V6 (GraphPad) using total binding as 100%.

### Monoamine oxidase A (MAO-A) assay

Compounds were tested for their capacity to modulate MAO-A activity within the MAO-Glo™ Assay (Promega, Cat.# V1402). In each well of a 96-well plate, 12.5 µl of 4X MAO Substrate solution (end-concentration in assay: 40 µM) were mixed with 12.5 µl of a 4X solution of the corresponding test compound (end-concentrations ranged from 10 µM to 1.37 nM). In the absence of a test compound (i.e. to gauge the total MAO-A activity), the corresponding volume of MAO Reaction Buffer (100 mM HEPES (pH 7.5), 5% glycerol) was added. To initiate the MAO reaction, 25 µl of 2X MAO-A enzyme solution (MAO-A human - recombinant, Sigma-Aldrich, Cat.# M7316; 1 µg MAO-A enzyme/25 µl MAO Reaction Buffer) were added per well and mixed briefly. For negative control reactions, 25µl of MAO Reaction Buffer were added. Thereafter the plate was incubated at room temperature for 1 hour, following which, 50 µl of reconstituted Luciferin Detection Reagent were added per well and mixed briefly. The plate was then incubated at room temperature for 20 minutes to generate and stabilize the luminescent signal. The luminescent signal was subsequently measured and recorded on the TopCountNXT™ luminescence counter (PerkinElmer). Data were analyzed and graphs were prepared using the Graphpad Prism 6 software.

### MAO-B assay

Compounds were tested for their capacity to modulate MAO-B activity within the MAO-Glo™ Assay (Promega, Cat.# V1402). In each well of a 96-well plate, 12.5 µl of 4X MAO Substrate solution (end-concentration in assay: 4 µM) were mixed with 12.5 µl of a 4X solution of the corresponding test compound (end-concentrations ranged from 10 µM to 1.37 nM). In the absence of a test compound (i.e. to gauge the total MAO-B activity), the corresponding volume of MAO-B Reaction Buffer (100 mM HEPES (pH 7.5), 5% glycerol, 10% dimethyl sulphoxide) was added. To initiate the MAO reaction, 25 µl of 2X MAO-B enzyme solution (MAO-B human - recombinant, Sigma-Aldrich, Cat.# M7441; 1 µg MAO-B enzyme/25 µl MAO-B Reaction Buffer) were added per well and mixed briefly. For negative control reactions, 25µl of MAO-B Reaction Buffer were added. Thereafter the plate was incubated at room temperature for 1 hour, following which, 50 µl of reconstituted Luciferin Detection Reagent were added per well and mixed briefly. The plate was then incubated at room temperature for 20 minutes to generate and stabilize the luminescent signal. The luminescent signal was subsequently measured and recorded on the TopCountNXT™ luminescence counter (PerkinElmer). Data were analyzed and graphs were prepared using the Graphpad Prism 6 software.

### Autoradiography using human brain tissue

18 micron thick frozen human brain slices and 6 micron thick human Formalin-fixed paraffin-embedded (FFPE) brain slices from the frontal and/or temporal lobes of AD patients were examined via autoradiography. Brain sections were equilibrated for at least 5 min in 1xPBS solution prior to use in the experiment. Each brain section was covered with a solution of the ¹⁸F labeled tracer (200Bq/µl, 500 µl) in 1xPBS. For blocking experiments with either the corresponding ¹⁹F-labeled compound (termed self-block) or other ¹⁹F-labeled compounds (termed cross-block), an excess of the blocking compound was mixed with the ¹⁸F-compound to yield an end concentration of 10 µM of the ¹⁹F-labeled compound. The brain sections were incubated in the tracer solution at room temperature for 1 h in a humidity chamber, drained thereafter and placed in a slide holder. The slides were then washed sequentially with 1×PBS for 1 min; 70% EtOH in 1×PBS for 2 min; 30% EtOH in 1×PBS for 1 min; and 1×PBS for 1 min. The slides were allowed to air-dry before being placed under Fuji imaging plates in imaging boxes for overnight exposure. The imaging plates were scanned using Fuji BAS-5000 software and the resulting images were analyzed and the signal was quantified using AIDA software.

Strong accumulation of activity was observed in gray matter areas. The specificity of the signal was confirmed by blocking with an excess of the corresponding non-radioactive compound. Additionally, selectivity was demonstrated by cross-blocking with a tracer binding to amyloid-beta. No competition of selected compounds from this invention by amyloid-beta-binding compounds was found.

### Brain PK studies in mice using PET imaging

NMRI mice (weight range 25-35 g) were injected intravenously with the 18F-labeled compounds. Up to 150 µL of 1xPBS, NaCl solution with 10%-15% EtOH or dilution medium (57% water for injections, 18% polyethylene glycol 400, 15% ethanol, 10% water) containing the 18F-labeled compound (2-10 MBq) were injected. Anesthesia with isoflurane and oxygen was induced before injection of the tracer and maintained during the image acquisition period. PET scans were performed using a SIEMENS INVEON small animal PET/CT scanner (Siemens, Knoxville, TN). PET acquisition was started immediately before the radioactive dose was injected into the animal through the tail vein. Images were generated as dynamic scans for 60 minutes. The peak uptake in the brain was set to 100% and washout curves were generated to evaluate the clearance of the activity from the normal brain. Brain wash-out curve for selected compound (**¹⁸F-5**) is illustrated in Figure 1.

**Table 6 Tau and MAO A/B binding data**

| **Examples** | **Tau binding (nM)** | **MAO A binding (nM)** | **MAO B binding (nM)** |
|---|---|---|---|
| 5 | 65 | 430 | > 1000 |
| 10 | 3 | 790 | > 1000 |
| 12 | 8 | 940 | > 1000 |

## Claims

1. A compound of formula (**I**): 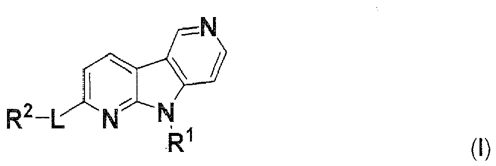 and all stereoisomers, racemic mixtures, pharmaceutically acceptable salts, hydrates, solvates and polymorphs thereof;
wherein
**R**¹ is -H or -CH₃,
**R**² is fluoro(hydroxy)alkylene,
**L** is 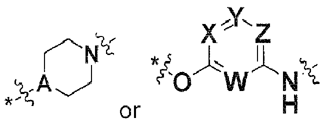
wherein the asterisk indicates the bond to **R**²;
**A** is N, 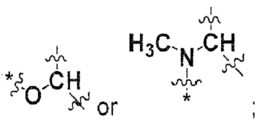
**W, X**, **Y**, and **Z** are selected from CH and N, with the proviso that the ring which includes **W, X, Y** and Z contains at most one nitrogen atom; and
**F** is [F-19]fluoro or [F-18]fluoro.

2. The compound according to claim 1, wherein **R²** is F-CH₂CH(OH)CH₂- or HO-CH₂CH(F)CH₂-.

3. The compound according to claim 1 or 2, wherein the compound is selected from a compound of formula (**IA**), (**IB**), (**IC**) and (**ID**): 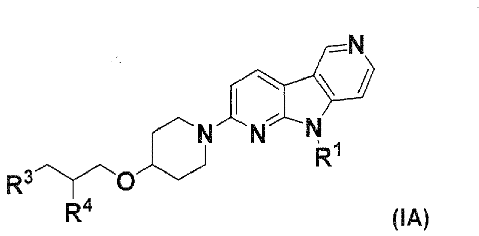 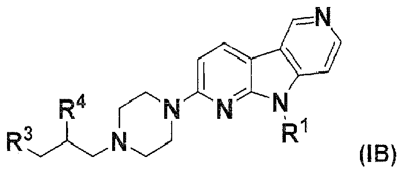 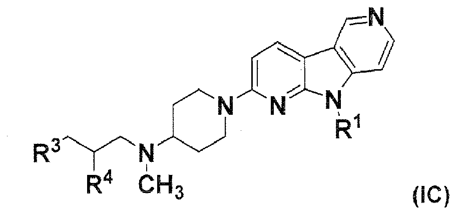 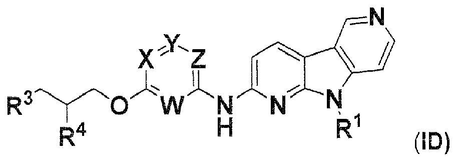 wherein **R**¹ is as defined in claim 1; and
wherein **R**³ is **OH** or **F** and wherein **R**⁴ is **OH** or **F,** with the proviso that if **R**³ is **OH, R**⁴ is **F,** or if **R**³ is **F, R**⁴ is **OH.**

4. A compound of formula (**II**): 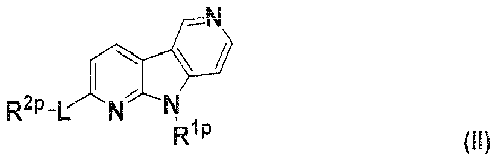 and all stereoisomers, racemic mixtures, pharmaceutically acceptable salts, hydrates, solvates and polymorphs thereof;
wherein
**R^{1p}** is -H, -CH₃ or **PG**¹, wherein **PG**¹ is an amine protecting group,
**R^{2p}** is **LG**(O**PG**²)alkylene,
**L** is 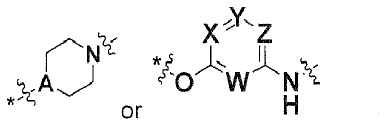
wherein the asterisk indicates the bond to **R**^{2p};
**PG²** is a hydroxy protecting group, and
**LG** is a leaving group.

5. A diagnostic composition comprising a compound according to any one of claims 1 to 3 and a pharmaceutically acceptable carrier, diluent, adjuvant or excipient.

6. The compound according to any one of claims 1 to 3 for use in diagnostics.

7. The compound according to any one of claims 1 to 3 for use in the imaging of tau aggregates, particularly positron emission tomography imaging of tau aggregates.

8. The compound according to any one of claims 1 to 3 for use in the diagnostics of a disorder associated with tau aggregates or in the diagnostics of a tauopathy.

9. The compound according to claims 8, wherein the disorder is selected from Alzheimer's disease (AD), familial AD, Creutzfeldt-Jacob disease, dementia pugilistica, Down's Syndrome, Gerstmann-Sträussler-Scheinker disease, inclusion-body myositis, prion protein cerebral amyloid angiopathy, traumatic brain injury, amyotrophic lateral sclerosis, Parkinsonism-dementia complex of Guam, non-Guamanian motor neuron disease with neurofibrillary tangles, argyrophilic grain disease, corticobasal degeneration, diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease, progressive subcortical gliosis, progressive supranuclear palsy (PSP), subacute sclerosing panencephalitis, tangle only dementia, postencephalitic Parkinsonism, myotonic dystrophy, tau panencephalopathy, AD-like with astrocytes, certain prion diseases (GSS with tau), mutations in LRRK2, Hallervorden-Spatz disease, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, and white matter tauopathy with globular glial inclusions; preferably Alzheimer's disease.

10. A method of imaging of tau aggregates, particularly positron emission tomography imaging of tau aggregates, wherein an effective amount of a compound according to any one of claims 1 to 3 is administered to a patient.

11. A method of diagnosing a disorder associated with tau aggregates or a tauopathy, wherein an effective amount of a compound according to any one of claims 1 to 3 is administered to a patient.

12. The method according to claim 11, wherein the disorder is selected from Alzheimer's disease (AD), familial AD, Creutzfeldt-Jacob disease, dementia pugilistica, Down's Syndrome, Gerstmann-Straussler-Scheinker disease, inclusion-body myositis, prion protein cerebral amyloid angiopathy, traumatic brain injury, amyotrophic lateral sclerosis, Parkinsonism-dementia complex of Guam, non-Guamanian motor neuron disease with neurofibrillary tangles, argyrophilic grain disease, corticobasal degeneration, diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease, progressive subcortical gliosis, progressive supranuclear palsy (PSP), subacute sclerosing panencephalitis, tangle only dementia, postencephalitic Parkinsonism, myotonic dystrophy, tau panencephalopathy, AD-like with astrocytes, certain prion diseases (GSS with tau), mutations in LRRK2, Hallervorden-Spatz disease, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, and white matter tauopathy with globular glial inclusions; preferably Alzheimer's disease.
